# EUROPEAN PATENT APPLICATION

(11) **EP 1 130 097 A1**
(43) Date of publication of application: **05.09.2001**
(21) Application number: 00403385.8
(22) Date of filing: 01.12.2000
(51) Int. Cl.: C12N 15/12, C07K 14/705, C07K 16/28, C12Q 1/68, G01N 33/68

(54) **Control of membrane traffic**

(30) Priority: 15.02.2000 EP 00400427
(71) Applicant: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: Galli, Thierry, 75005 Paris (FR); Louvard, Daniel, 92330 Suresnes (FR); Martinez, Sonia, 75005 Paris (FR)
(74) Representative: Peaucelle, Chantal

(57) **Abstract**

The present invention relates to the control of membrane traffic inside cells, such as those involving fusion events and in particular those involving exocytic events. It more particularly relates to N-terminal fragments of TeNT-insensitive VAMP, and to TeNT-insensitive VAMP deleted from such fragments, and to the biological applications of such products, notably for controlling TeNT-resistant pathways such as neurite outgrowth and cell motility.

SEQUENCE LISTING

<110> Institut Curie
      C.N.R.S.

<120> CONTROL OF MEMBRANE TRAFFIC.

<130> 1100EP2000

<140>
<141>

<160> 22

<170> PatentIn Ver. 2.1

<210> 1
<211> 360
<212> DNA
<213> Homo sapiens

<400> 1
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gccatgaata gcgagttctc aagtgtctta gctgcacagc tgaagcatca ctctgagaat 360

<210> 2
<211> 120
<212> PRT
<213> Homo sapiens

<400> 2
Met Ala Ile Leu Phe Ala Val Val Ala Arg Gly Thr Thr Ile Leu Ala
  1               5                  10                  15

Lys His Ala Trp Cys Gly Gly Asn Phe Leu Glu Val Thr Glu Gln Ile
             20                  25                  30

Leu Ala Lys Ile Pro Ser Glu Asn Asn Lys Leu Thr Tyr Ser His Gly
         35                  40                  45

Asn Tyr Leu Phe His Tyr Ile Cys Gln Asp Arg Ile Val Tyr Leu Cys
     50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn
        115                 120

<210> 3
<211> 357
<212> DNA
<213> Homo sapiens

<400> 3
atgaatagcg agttctcaag tgtcttagct gcacagctga agcatcactc tgagaataag 60
ggcctagaca aagtgatgga gactcaagcc caagtggatg aactgaaagg aatcatggtc 120
agaaacatag atctggtagc tcagcgagga gaaagattgg aattattgat tgacaaaaca 180
gaaaatcttg tggattcttc tgtcaccttc aaaactacca gcagaaatct tgctcgagcc 240
atgtgtatga agaacctcaa gctcactatt atcatcatca tcgtatcaat tgtgttcatc 300
tatatcattg tttcacctct ctgtggtgga tttacatggc caagctgtgt gaagaaa    357

<210> 4
<211> 119
<212> PRT
<213> Homo sapiens

<400> 4
Met Asn Ser Glu Phe Ser Ser Val Leu Ala Ala Gln Leu Lys His His
1               5                   10                  15

Ser Glu Asn Lys Gly Leu Asp Lys Val Met Glu Thr Gln Ala Gln Val
            20                  25                  30

Asp Glu Leu Lys Gly Ile Met Val Arg Asn Ile Asp Leu Val Ala Gln
        35                  40                  45

Arg Gly Glu Arg Leu Glu Leu Leu Ile Asp Lys Thr Glu Asn Leu Val
    50                  55                  60

Asp Ser Ser Val Thr Phe Lys Thr Thr Ser Arg Asn Leu Ala Arg Ala
65                  70                  75                  80

Met Cys Met Lys Asn Leu Lys Leu Thr Ile Ile Ile Ile Ile Val Ser
                85                  90                  95

Ile Val Phe Ile Tyr Ile Ile Val Ser Pro Leu Cys Gly Gly Phe Thr
            100                 105                 11[?]

Trp Pro Ser Cys Val Lys Lys
        115

<210> 5
<211> 801
<212> DNA
<213> Homo sapiens

<400> 5
gggcctctag atgcatgctc gagcggccgc cagtgtgatg gatatctgca gaattcggct 60
tagactgaag ccatggcgat tctttttgct gttgttgcca gggggaccac tatccttgcc 120
aaacatgctt ggtgtggagg aaacttcctg gaggtgacag agcagattct ggctaagata 180
ccttctgaaa ataacaaact aacgtactca catggcaatt atttgtttca ttacatctgc 240
caagacagga ttgtatatct ttgtatcact gatgatgatt ttgaacgttc ccgagccttt 300
aatttctgaa atgagataaa gaagaggttc cagactactt acggttcaag agcacagaca 360
gcacttccat atgccatgaa tagcgagttc tcaagtgtct tagctgcaca gctgaagcat 420
cactctgaga ataagggcct agacaaagtg atggagactc aagcccaagt ggatgaactg 480
aaaggaatca tggtcagaaa catagatctg gtagctcagc gaggagaaag attggaatta 540
ttgattgaca aaacagaaaa tcttgtggat tcttctgtca ccttcaaaac taccagcaga 600
aatcttgctc gagccatgtg tatgaagaac ctcaagctca ctattatcat catcatcgta 660
tcaattgtgt tcatctatat cattgtttca cctctctgtg gtggatttac atggccaagc 720
tgtgtgaaga aatagaagcc gaattccagc acactggcgg ccgttactag tggatccgag 780
ctcggtacca agcttgatgc a                                           801

<210> 6
<211> 220
<212> PRT
<213> Homo sapiens

<400> 6
Met Ala Ile Leu Phe Ala Val Val Ala Arg Gly Thr Thr Ile Leu Ala
1               5                   10                  15

Lys His Ala Trp Cys Gly Gly Asn Phe Leu Glu Val Thr Glu Gln Ile
            20                  25                  30

Leu Ala Lys Ile Pro Ser Glu Asn Asn Lys Leu Thr Tyr Ser His Gly
        35                  40                  45

Asn Tyr Leu Phe His Tyr Ile Cys Gln Asp Arg Ile Val Tyr Leu Cys

50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn Lys Gly Leu Asp Lys Val Met Glu
        115                 120                 125

Thr Gln Ala Gln Val Asp Glu Leu Lys Gly Ile Met Val Arg Asn Ile
    130                 135                 140

Asp Leu Val Ala Gln Arg Gly Glu Arg Leu Glu Leu Leu Ile Asp Lys
145                 150                 155                 160

Thr Glu Asn Leu Val Asp Ser Ser Val Thr Phe Lys Thr Thr Ser Arg
                165                 170                 175

Asn Leu Ala Arg Ala Met Cys Met Lys Asn Leu Lys Leu Thr Ile Ile
            180                 185                 190

Ile Ile Ile Val Ser Ile Val Phe Ile Tyr Ile Ile Val Ser Pro Leu
        195                 200                 205

Cys Gly Gly Phe Thr Trp Pro Ser Cys Val Lys Lys
    210                 215                 220

<210> 7
<211> 564
<212> DNA
<213> Homo sapiens

<400> 7
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gccatgaata gcgagttctc aagtgtctta gctgcacagc tgaagcatca ctctgagaat 360
aagggcctag acaaagtgat ggagactcaa gcccaagtgg atgaactgaa aggaatcatg 420
gtcagaaaca tagatctggt agctcagcga ggagaaagat tggaattatt gattgacaaa 480
acagaaaatc ttgtggattc ttctgtcacc ttcaaaacta ccagcagaaa tcttgctcga 540

gccatgtgta tgaagaacct caag                                          564

<210> 8
<211> 188
<212> PRT
<213> Homo sapiens

<400> 8
Met Ala Ile Leu Phe Ala Val Val Ala Arg Gly Thr Thr Ile Leu Ala
1               5                   10                  15

Lys His Ala Trp Cys Gly Gly Asn Phe Leu Glu Val Thr Glu Gln Ile
            20                  25                  30

Leu Ala Lys Ile Pro Ser Glu Asn Asn Lys Leu Thr Tyr Ser His Gly
        35                  40                  45

Asn Tyr Leu Phe His Tyr Ile Cys Gln Asp Arg Ile Val Tyr Leu Cys
    50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn Lys Gly Leu Asp Lys Val Met Glu
        115                 120                 125

Thr Gln Ala Gln Val Asp Glu Leu Lys Gly Ile Met Val Arg Asn Ile
    130                 135                 140

Asp Leu Val Ala Gln Arg Gly Glu Arg Leu Glu Leu Leu Ile Asp Lys
145                 150                 155                 160

Thr Glu Asn Leu Val Asp Ser Ser Val Thr Phe Lys Thr Thr Ser Arg
                165                 170                 175

Asn Leu Ala Arg Ala Met Cys Met Lys Asn Leu Lys
            180                 185

<210> 9
<211> 27

<212> DNA
<213> Artificial Sequence

<220>
<223> Description of Artificial Sequence:PCR PRIMERS

<400> 9
atggcgattc tttttgctgt tgttgcc                                          27


<210> 10
<211> 27
<212> DNA
<213> Artificial Sequence

<220>
<223> Description of Artificial Sequence:PCR PRIMERS

<400> 10
ctatttcttc acacagcttg gccatgt                                          27


<210> 11
<211> 27
<212> DNA
<213> Artificial Sequence

<220>
<223> Description of Artificial Sequence:PCR PRIMERS

<400> 11
atggcgattc tttttgctgt tgttgcc                                          27


<210> 12
<211> 27
<212> DNA
<213> Artificial Sequence

<220>
<223> Description of Artificial Sequence:PCR PRIMERS

<400> 12
cttattctca gagtgatgct tcagctg                                          27


<210> 13
<211> 27

<212> DNA
<213> Artificial Sequence

<220>
<223> Description of Artificial Sequence:PCR PRIMERS

<400> 13
atggcgattc tttttgctgt tgttgcc                                          27


<210> 14
<211> 32
<212> DNA
<213> Artificial Sequence

<220>
<223> Description of Artificial Sequence:PCR PRIMERS

<400> 14
atcctacttg aggttcttca tacacatggc tc                                    32


<210> 15
<211> 27
<212> DNA
<213> Artificial Sequence

<220>
<223> Description of Artificial Sequence:PCR PRIMERS

<400> 15
atgaatagcg agttctcaag tgtctta                                          27


<210> 16
<211> 27
<212> DNA
<213> Artificial Sequence

<220>
<223> Description of Artificial Sequence:PCR PRIMERS

<400> 16
ctatttcttc acacagcttg gccatgt                                          27


<210> 17
<211> 27

<212> DNA
<213> Artificial Sequence

<220>
<223> Description of Artificial Sequence:PCR PRIMERS

<400> 17
atgtcggcta ccgctgccac cgtcccg                                    27


<210> 18
<211> 27
<212> DNA
<213> Artificial Sequence

<220>
<223> Description of Artificial Sequence:PCR PRIMERS

<400> 18
ttaagagctg aagtaaacta tgatgat                                    27


<210> 19
<211> 303
<212> DNA
<213> Homo sapiens

<400> 19
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gcc                                                              303


<210> 20
<211> 66
<212> PRT
<213> Homo sapiens

<400> 20
Met Ala Ala Val Val Ala Arg Gly Thr Thr Ala Lys His Ala Trp Cys
1               5                   10                  15

Gly Gly Asn Val Thr Ala Lys Ser Asn Asn Lys Thr Tyr Ser His Gly
            20                  25                  30

Asn Tyr His Tyr Cys Asp Arg Val Tyr Cys Thr Asp Asp Asp Arg Ser
        35                  40                  45

Arg Ala Asn Asn Lys Lys Arg Thr Thr Tyr Gly Ser Arg Ala Thr Ala
    50                  55                  60

Tyr Ala
65

<210> 21
<211> 27
<212> DNA
<213> Artificial Sequence

<220>
<223> Description of Artificial Sequence:PCR primer

<400> 21
ggatcctttc ttcacacagc ttggcca 27

<210> 22
<211> 27
<212> DNA
<213> Artificial Sequence

<220>
<223> Description of Artificial Sequence:PCR primer

<400> 22
ctatttcttc acacagcttg gccatgt 27

## Description

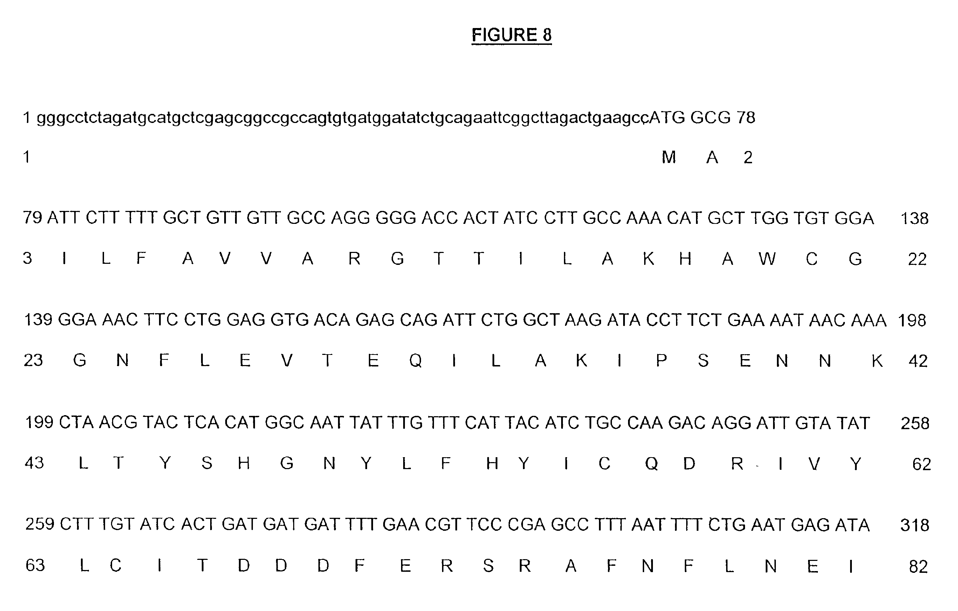

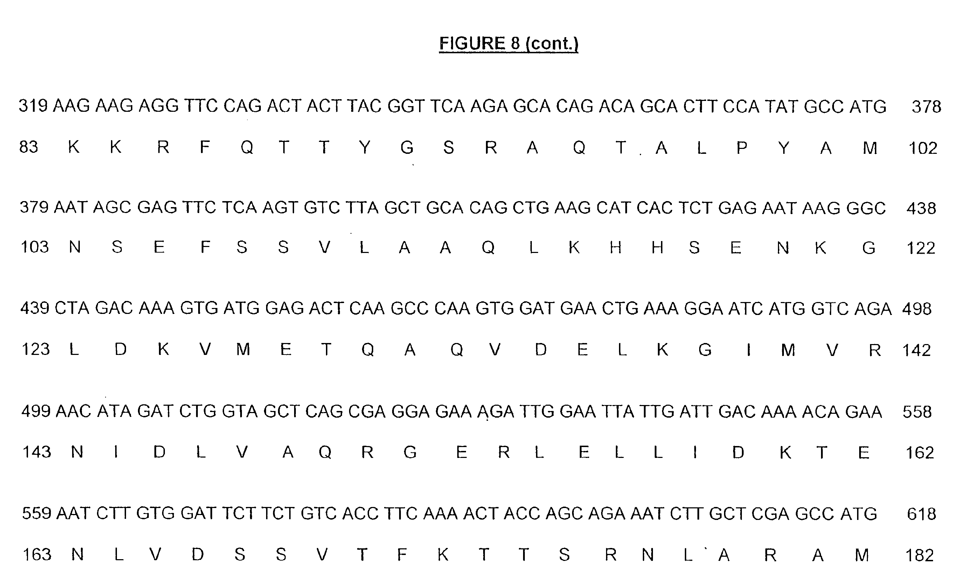

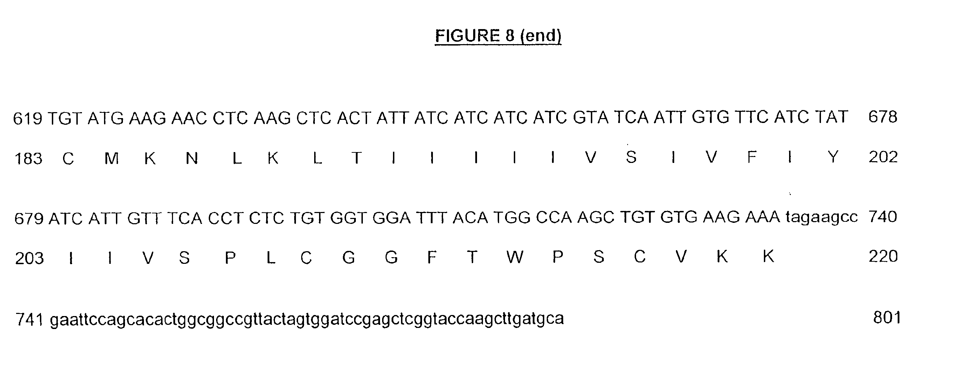

The present invention relates to the control of membrane traffic inside cells, such as those involving fusion events and in particular those involving exocytic events. It more particularly relates to the positive and negative regulation of any tetanus neurotoxin (TeNT) -resistant pathway inside a cell, such as any pathway involving the activity of a TeNT-insensitive VAMP (vesicular-associated membrane protein). The present invention indeed provides with new products and new means for controlling such a membrane traffic inside a cell, and notably for controlling any cell activity involving a TeNT-insensitive VAMP such as TI-VAMP (tetanus neurotoxin-insensitive vesicle-associated membrane protein).
Many different VAMP have been described in the prior art. These VAMP are receptors expressed by cell vesicles, and are known to be involved in exocytic events. But very little was known on how to control the activity of TeNT-insensitive VAMP, and of TI-VAMP in particular.
The present invention provides with new products and means solving this problem, and further gives for the first time the demonstration that such new products and means are efficient in controlling membrane traffic in non-epithelial cells, and notably in neuronal cells and in tumor cells.
The present invention thus encompasses any isolated polypeptide , the sequence of which corresponds to a sequence selected from the group consisting of the N-terminal domain sequences of any TeNT-insensitive VAMP such as TI-VAMP, and the conservative fragments and variants thereof. Such isolated polypeptides do not comprise any coiled coil motif.
It more particularly relates to any isolated polypeptide , the sequence of which corresponds to a sequence selected from the group consisting of a sequence corresponding to SEQ ID N°2, a sequence corresponding to SEQ ID N°20, the sequences corresponding to any conservative fragment of SEQ ID N°2, the sequences corresponding to any conservative fragment of SEQ ID N°20, the sequences corresponding to any conservative variant of SEQ ID N°2, the sequences corresponding to any conservative variant of SEQ ID N°20.
These polypeptides will be referred to herein as the Nter polypeptides.
SEQ ID N°2 refers to an isolated polypeptide , the sequence of which corresponds to the first 120 amino acids (M¹-N¹²⁰ in Figure 8) of human normal TI-VAMP (SEQ ID N°6). SEQ ID N°2 is also referred to as Nter in the below examples. SEQ ID N°20 refers to an isolated polypeptide , the sequence of which corresponds to the first 101 amino acids (M¹-A¹⁰¹ on Figure 8) of human normal TI-VAMP (SEQ ID N°6).

As these aa sequences, their corresponding polynucleotidic sequences, and the conservative fragment sequences thereof are from normal human origin, they and their human variants are considered as corresponding to one of the best modes of the invention. Whereas the complete sequence of TI-VAMP has been previously described, N-terminal domain (*i*.*e*. N-terminal region which excludes any coiled coil motif) had no known function up to the present invention. As there was no aim at it, the skilled person would then not have seriously encompassed to produce a polypeptide limited to such a N-terminal domain, or to produce a polypeptide limited to the coiled coils plus C-terminal region of TI-VAMP. The present invention describes for the first time a biological function for these polypeptides : they are capable of regulating a membrane traffic, and in particular TeNT-resistant pathways. According to a further remarkable aspect, they are capable of exerting these capacities on neuronal maturation and/or differentiation (neurite outgrowth) and on the motility of tumor cells.
By « conservative » product (polypeptide or polynucleotidic fragment, variant polypeptide or polynucleotide), it is meant in the present application that this product is capable of showing under physiological conditions at least one of the biological properties shown by a « parent » product (SEQ ID N°2 -also referred to as Nter in the below examples -, and SEQ ID N°20 -from M¹ to A¹⁰¹ on figure 8).
Such biological properties notably include the capacity of inhibiting a membrane traffic pathway inside a cell, the capacity of inhibiting a function involving at least one TeNT-insensitive pathway, the capacity of inhibiting the activity of a TeNT-insensitive VAMP such as TI-VAMP, the capacity of inhibiting a function involving a fusion function or an exocytose function of a cell, a capacity of inhibiting neurite outgrowth, a capacity of inhibiting the motility of cells such as metastasis-forming cells (tumor cells). Any cell enabling the skilled person to perform the desired assay is appropriate. Preferred cells are cells of animal or human origin, including cell lines. Cells of special interest for industrial applications namely include neuronal cells and tumor cells, from primary cultures, as well as from cell lines such a PC12 cells for neuronal cells, or MDCK, HeLAa, CACO-2, NIH-3-T3 cells for tumor cells.
By physiological conditions, it is herein meant *in vivo* conditions, or *in vitro* conditions mimicking the *in vivo* ones ; typically, appropriate physiological conditions comprise conditions of medium composition, atmosphere, pH which are adequate to the cells that may be involved in the assay, and notably to animal or human cells.
By a « variant » product, it is herein meant any product which corresponds to the « parent » product after one or several of its elementary components (aminoacid, or when the case arises, nucleotide) has (have) been deleted and/or inverted and/or substituted, in so far this variant product has retained at least one of the biological properties shown by the « parent » product as above-defined. When reference is made to any amino acid or polypeptide deletion/inversion/substitution, it has to be considered in the context of the universal genetic code and its redundancies. A variant product thus notably encompasses any product , the sequence of which shows with the parent product (SEQ ID N°2 or N°20 in the case of polypeptides; SEQ ID N°1 or SEQ ID N°19 for their respective DNA sequences), over the entire length of this parent product sequence, an homology of at least about 50%, particularly of at least about 60%, more particularly of at least about 70%, preferably of at least about 80%, more preferably of at least about 90%, the most preferred being of at least about 95%. A variant product also encompasses any conservative fragments of such products. While the invention is more particularly illustrated for human cells, variant products which can be found in other cell types or in other species, and notably among animals, are thus encompassed by the present invention.
By "TeNT-resistant" or "TeNT-insensitive pathway or receptor, the present application means that said pathway or said receptor is still active when the cell wherein said pathway takes places or said receptor is expressed is placed into contact with tetanus neurotoxin under exposure to the TeNT produced by the clostridium tetanii in the case of sensitive cells such as neurons, or of transfection of the cDNA coding for the light chain of TeNT in all cases.
In the present application, the expressions "inhibiting", "stimulating" are meant as statistically significant negative or positive difference, and thus encompasses "blocking" and, respectively, "inducing".
According to a complementary and corresponding aspect, the invention also relates to any isolated polypeptide , the sequence of which corresponds to a sequence selected from the group consisting of the TeNT-insensitive VAMP sequences, such as TI-VAMP sequence, deleted from their respective N-terminal domains, provided that the resulting "deleted" polypeptide is still capable of showing an activity of the coiled coil type (*i*.*e*. the SNARE motif activity is not disrupted), namely is still capable of binding to at least one of the target SNARE (target Soluble N-ethylmaleide-sensitive fusion protein Attachment Receptor) of the corresponding complete VAMP (t-SNARE of TI-VAMP namely comprise SNAP 25, SNAP23, syntaxinl, syntaxin3). Coiled coils of VAMP can be predicted by computer programs such as COILS. These polypeptides will be referred to herein as the « deleted » polypeptides (also referred to as △Nter-TI-VAMP in the below examples). The invention more particularly relates to polypeptides , the sequence of which corresponds to SEQ ID N°6 of which N-terminal domain has been deleted from at least one Nter polypeptide according to the invention. Examples of such "deleted polypeptides" include those , the sequence of which corresponds to a sequence selected from the group consisting of SEQ ID N°4, the sequences corresponding to any conservative variant of SEQ ID N°4, and the sequences corresponding to any conservative fragment of SEQ ID N°4.
SEQ ID N°6 refers to the complete amino acid sequence of human TI-VAMP (see figure 8).
SEQ ID N°4 refers to the amino acid sequence of an isolated polypeptide , the sequence of which corresponds to the amino acid sequence of human TI-VAMP deleted from the first 101 N-terminal amino acids (*i*.*e*. it corresponds to M¹⁰² to the end of SEQ ID N°6).
As SEQ ID N°4, its corresponding polynucleotidic sequence (SEQ ID N°3), and the conservative fragments thereof are from normal human origin, they and their human variants are considered as one of the best modes of the invention.
The biological properties shown by a polypeptide of SEQ ID N°4 correspond to reversed properties of SEQ ID N°2 or 20. They notably include the capacity of stimulating a membrane traffic pathway inside a cell, the capacity of stimulating a function involving at least one TeNT-resistant pathway, the capacity of stimulating the activity of a TeNT-insensitive VAMP such as TI-VAMP, the capacity of stimulating a function involving a fusion function or an exocytose function of a cell, a capacity of stimulating neurite outgrowth, a capacity of stimulating the motility of cells such as metastasis-forming cells. « variant » is as above-defined.

Both the Nter and the "deleted" polypeptides of the invention are advantageously associated to any product enabling their passage inside a cell, such as an animal cell, a human cell, a plant cell. an eucaryotic cell, a protiste.
So as to make it penetrate into a cell, a polypeptide of the invention can be *e*.*g*. chemically modified, for examples by esterification by addition of a lipidic tail, and/or can be associated to a carrier-delivery system such as liposomes.

The invention further provides with products derived from the Nter polypeptides of the invention. It thus relates to any product selected from the group consisting of the monoclonal antibodies capable of binding to a polypeptide according to the invention, and the Fab, F(ab')₂, CDR fragments thereof. These products will be referred to as the "binding" products of the invention. Production of monoclonal antibodies is of common knowledge to the skilled person (notably : Köhler and Milstein procedure). Such monoclonal antibodies advantageously do not bind to a « deleted » polypeptide of the invention. The invention more particularly encompasses those products which are capable of inhibiting under physiological conditions at least one of the biological properties a Nter polypeptide according to the invention can show.
Such biological properties of a Nter polypeptide namely comprise the inhibition of the formation of complexes between a TeNT-insensitive VAMP such as TI-VAMP, and a target SNARE *(e.g.* SNAP25, SNAP23, syntaxin1, syntaxin3 for TI-VAMP).

According to further aspect of the invention, sharing corresponding features with the polypeptides of the invention, there are provided polynucleotides, the sequence of which codes for the Nter polypeptides according to the invention, polynucleotides , the sequence of which codes for the "deleted" polypeptides according to the invention, and polynucleotides , the sequence of which codes for any binding product according to the invention.

As Nter polynucleotides, the invention more particularly relates to any isolated polynucleotide , the sequence of which corresponds to a sequence chosen , the sequence of which corresponds to a sequence selected from the group consisting of a sequence corresponding to SEQ ID N°1, a sequence corresponding to SEQ ID N°19, the sequence corresponding to any conservative fragment of SEQ ID N°1, the sequence corresponding to any conservative variant of SEQ ID N°1, the sequence corresponding to any conservative fragment of SEQ ID N°19, the sequence corresponding to any conservative variant of SEQ ID N°19.
SEQ ID N°1 refers to the DNA sequence coding for SEQ ID N°2 (first N-terminal 120 aa of TI-VAMP, *i*.*e*. from M¹ to N¹²⁰, that is to say from position 73 to position 432 on the TI-VAMP DNA Sequence, see figure 8).
SEQ ID N°19 refers to the DNA sequence coding for SEQ ID N°20 (first N-terminal 101 aa of TI-VAMP, *i*.*e*. from M¹ to position 101, that is to say from position 73 to position 375 on the TI-VAMP DNA sequence, see figure 8).
As "deleted" polynucleotides, the invention more particularly relates to any isolated polynucleotide , the sequence of which corresponds to a sequence selected from the group consisting of the SEQ ID N°5 sequences of which 5' domain has been deleted from at least one Nter polynucleotide according to the invention. The "deleted" polynucleotides of the invention namely comprise any isolated polynucleotide , the sequence of which corresponds to a sequence selected from the group consisting of a sequence corresponding to SEQ ID N°3, the sequences corresponding to any conservative fragment of SEQ ID N°3, the sequences corresponding to any conservative variant of SEQ ID N°3.
SEQ ID N°5 refers to the complete DNA sequence of human TI-VAMP (see figure 8).
SEQ ID N°3 refers to the DNA sequence of an isolated polynucleotide , the sequence of which corresponds to a sequence coding for M¹⁰² to the end of SEQ ID N°6, that is to say corresponds to a position 376-732 DNA fragment of SEQ ID N°5.

All of these polynucleotides are particularly useful for transfection into a cell such as an animal cell, a human cell, a cell line, a plant cell, an eucaryotic cell, a protist. They can be directly electroporated into a cell such as a neuronal or dendritic cell (see examples below). When appropriate, transfection vectors, such as plasmids or retrovirus, which comprise at least one polynucleotide according to the invention, can alternatively be constructed by the skilled person. Preferably, said at least one polynucleotide is comprised in said transfection vector in a coding position.
Appropriate promotors namely comprise those of cytomegalovirus or of TI-VAMP himself (Synaptobrevin like gene 1, Matarazzo *et al*. Gene 1999, 240:233-238).
When the transfection vector comprises at least one Nter polynucleotide of the invention, it is herein referred to as "Nter" transfection vector.
When the transfection vector comprises at least one "deleted" polynucleotide of the invention, it is herein referred to as "deleted" transfection vector.] Examples of such vectors and of such transfection notably comprise viral vectors, retroviral vectors, adenoviral vectors, adeno-associated vectors (Aav), lentivirus, herpes virus, plasmids, or the like. Several of them are illustrated in the below examples. Advantageous transfection vectors namely comprise recombinant adeno-associated virus (see example 4 below ; and see also Berns and Bohensky 1987 Advances in Virus Research (Academic Press, Inc.) 32: 243-307 ; Kaplitt et al. 1994, Nature Genetics 8: 148-154 ; US 5,175,414 ; US 5,139,941; Vincent et al. 1990 Vaccines 90 (Cold Spring Harbor Laboratory Press) ; Kotin et al. 1994 Human Gene Therapy 5: 793-801; Du et al. 1996, Gene Ther. 3(3): 254-261; Slack and Miller 1996, Curr. Opin. Neurobiol. 6(5): 576-583).
Recombinant adeno-associated virus comprising at least one Nter polynucleotide or at least one "deleted" polynucleotide of the invention will be herein referred to as the Nter adeno-associated virus of the invention, and to the "deleted" adeno-associated virus of the invention, respectively. They are particularly useful for transfecting cells such as neuronal cells. They represent useful agents for gene therapy, or anti-sense therapy. The present invention encompasses their use for the production of a pharmaceutical composition or a drug of the invention (see below), and also encompasses the pharmaceutical compositions and drugs resulting therefrom.
The invention further encompasses any cell that has been genetically engineered cell so as to comprise at least one product selected from :
- the group consisting of the Nter polynucleotides according to the invention, the Nter transfection vectors according to the invention (and notably the Nter adeno-associated virus of the invention), this group being referred to as the "Nter" cells of the invention, or
- the group consisting of the "deleted" polynucleotides according to the invention, the transfection vectors according to the invention (and notably the "deleted" adeno-associated virus of the invention), this group being referred to as the "deleted" cells of the invention.
Preferred Nter and "deleted" cells of the invention produce Nter, or respectively, "deleted" polypeptides according to the invention.
Preferred cells are protist cells, eucaryotic cells, human cells, animal cells such as animal ovary cells, human or animal neuronal cells, dendritic cells, tumor cells. The invention notably encompasses any dendritic or neuronal cell which has been transfected with an adeno-associated virus of the invention.
According to a further complementary aspect of the invention, there are provided new products enabling the isolation of the polynucleotides of the invention.
The invention indeed encompasses any pair of oligonucleotides characterized in that it is capable under standard PCR conditions to amplify at least one Nter polynucleotide according to the invention.
Such oligonucleotide pairs namely comprise the SEQ ID N°11, SEQ ID N°12 pair (see example 1 below).

The invention correspondingly also encompasses any pair of oligonucleotides characterized in that it is capable under standard PCR conditions to amplify at least one "deleted" polynucleotide according to the invention.
Such oligonucleotide pairs namely comprise the SEQ ID N°15, SEQ ID N°16 pair (see example 1 below).
By standard PCR conditions, it is herein meant PCR conditions that enable the skilled person to amplify from a polynucleotidic population the desired polynucleotide at the desired purity or specificity. The adjustment of such conditions are of common knowledge. Examples of appropriate conditions include the calculation of the melting temperature of the primers according to common knowledge in the art, and a choice of MgCl₂ concentration so as to establish the desired stringency.
These oligonucleotidic pairs of the invention allow the isolation of Nter polynucleotides according to the invention, and respectively "deleted" polynucleotides according to the invention. This isolation can be performed from a polynucleotide population extracted from cells such as human cells. A standard way to proceed is to place at least one oligonucleotidic pair according to the invention into contact with such a polynucleotide population under conditions appropriate to PCR amplification as above-defined. This method of the invention namely allows the skilled person to obtain any desired variant and/or fragment of Nter polynucleotides and "deleted" polynucleotides of the invention. It further enables to isolate mutant non-functional forms of human or animal Nter and "deleted" polynucleotides of the invention.
The present invention thus encompasses any isolated polynucleotide which is such as obtained by using on a polynucleotide population at least one pair of oligonucleotides according to the invention. Appropriate polynucleotide populations namely comprise those extracted from human or animal cells, from human or animal normal cells, from human or animal pathological cells, from human or animal nerve or neuronal cells, from human or animal tumor cells. An easy way to proceed is via PCR.

Once such polynucleotides are isolated, it is of common knowledge to the skilled person to deduce from them the amino acid sequences that correspond to them.
According to aspect of particular interest for industrial application, the invention provides with pharmaceutical compositions which comprise at least one product selected from the group consisting of the Nter polypeptides according to the invention, the Nter polynucleotides according to the invention, the Nter transfection vectors according to the invention (and notably the Nter adeno-associated virus of the invention), and the Nter cells according to the invention. Such a pharmaceutical composition may notably be a drug, which can be administered to a human or to an animal. In this case, the drug of the invention may comprise said at least one product in a quantity appropriate for inhibiting a cell membrane traffic, and/or a function involving a TeNT-insensitive pathway, and/or a function involving a TeNT-resistant VAMP such as TI-VAMP, and/or a cell fusion or cell exocytic function, and/or the formation of complexes involving a TeNT-insensitive VAMP such as TI-VAMP, and/or a cell undesired motility such as the motility of cells susceptible of forming metastasis. Pharmaceutical compositions of the invention thus notably comprise anti-tumor drugs. It also relates to the use of such products for the production of such a pharmaceutical composition.

According to another aspect of particular interest for industrial application, the invention provides with pharmaceutical composition comprising at least one product selected from the group consisting of the "deleted" polypeptides according to the invention, the "deleted" polynucleotides according to the invention, the "deleted" transfection vectors according to the invention (and notably the "deleted" adeno-associated virus of the invention), and the "deleted" cells according to the invention. Such a pharmaceutical composition may notably be a drug, which can be administered to a human or an animal. In this case, the drug of the invention may comprise said at least one product in a quantity appropriate for stimulating a cell membrane traffic, and/or a function involving a TeNT-resistant pathway, and/or a function involving a TeNT-insensitive VAMP such as TI-VAMP, and/or a cell fusion or a cell exocytic function, and/or the formation of complexes involving a TeNT-insensitive VAMP such as TI-VAMP, and/or a neurite outgrowth (axonal and/or dendritic outgrowth), and/or a neuronal maturation, and/or a neuronal differentiation, and/or appropriate for stimulating memory and/or learning. Pharmaceutical compositions of the invention thus notably comprise drugs intended for the therapy and/or palliation and/or prevention of spinal cord trauma.
It also relates to the use of such products for the production of such a pharmaceutical composition.
The pharmaceutical compositions and drugs of the invention may further comprise any additive, co-agent, buffer appropriate to the application for which it is intended. The formulation of such compositions and drugs can be chosen and adjusted by the skilled person in function of the precise reactive agent chosen (polypeptide/polynucleotide/transfection vector/cell), in function of the additives co-agents and/or buffer that have been chosen, and has to take into account the administration route that is desired (topical, oral, injection, subcutaneous, implant, patch, spray, transfection, etc.).

The invention also provides with new means, making use of the new products of the invention, and sharing with them the same or corresponding feature of the inhibition function of which is capable the N-terminal domain of a TeNT-insensitive VAMP such as TI-VAMP.
The invention notably provides with a method for identifying a pharmaceutical agent capable of stimulating a cell function selected from the group consisting of the cell functions involving a membrane traffic, the cell functions involving a TeNT-resistant pathway, the cell functions involving the formation of complexes with a TeNT-insensitive VAMP such as TI-VAMP, the cell functions involving at least one TeNT-insensitive VAMP such as TI-VAMP, the cell functions involving a fusion, or an exocytic event, the cell functions involved in neurite outgrowth, in neuronal maturation, in neuronal differentiation, in neuronal or dendritic viability, in memory ability, in learning capacity, characterized in that it comprises at least one step selected from the group consisting of:
- the identification of an agent that is capable under physiological conditions of blocking or diminishing the inhibition effect that is observed when the cytoplasm of said cell is placed into contact with at least one product selected from the group consisting of the Nter polypeptides according to the invention, the Nter polynucleotides according to the invention, the Nter transfection vectors according to the invention, the Nter adeno-associated virus of the invention, the Nter cells according to the invention, under conditions appropriate to cell membrane trafficking,
- the identification of an agent that is capable under physiological conditions to compete with a "binding" product according to the invention for binding to at least one Nter polypeptide of the invention,
- the identification of an agent that is capable under physiological conditions of stimulating the formation of complexes involving at least one "deleted" polypeptide according to the invention, and at least one t-SNARE (such as SNAP25, SNAP23, syntaxin1, syntaxin3 for TI-VAMP),
- the identification of an agent that is capable under physiological conditions of exerting an additional or synergic effect on the cell function that is observed when the cytoplasm of said cell is placed into contact with at least one product selected from the group consisting of the "deleted" polypeptides according to the invention, the "binding" products according to the invention, the "deleted" polynucleotides according to the invention, the "deleted" transfection vectors according to the invention, the "deleted" adeno-associated virus of the invention, the "deleted" cells according to the invention, under conditions appropriate to cell membrane trafficking,
- the identification of an agent that is capable under physiological conditions of preventing the formation of complexes between compounds which comprise at least one Nter polypeptide of the invention, or of disrupting or destabilizing such complexes.

The invention also provides with a method for identifying a pharmaceutical agent capable of inhibiting a cell function selected from the group consisting of the cell functions involving a membrane traffic, the cell functions involving a TeNT-resistant pathway, the cell functions involving the formation of complexes with a TeNT-insensitive VAMP such as TI-VAMP, the cell functions involving at least one TeNT-insensitive VAMP such as TI-VAMP, the cell functions involving a fusion, or an exocytic event, the cell functions involved in cell motility, the cell functions involved in the formation of metastasis, characterized in that it comprises at least one step selected from the group consisting of:
- the identification of an agent that is capable under physiological conditions of stimulating the inhibition effect that is observed when the cytoplasm of said cell is placed into contact with at least one product selected from the group consisting of the Nter polypeptides according to the invention, the Nter polynucleotides according to the invention, the Nter transfection vectors according to the invention, the Nter adeno-associated virus of the invention, the Nter cells according to the invention, under conditions appropriate to cell membrane trafficking,
- the identification of an agent that is capable under physiological conditions of inhibiting the formation of complexes involving at least one "deleted" polypeptide according to the invention, and at least one t-SNARE (such as SNAP25, SNAP23, syntaxin1, syntaxin3 for TI-VAMP),
- the identification of an agent that is capable under physiological conditions of exerting an inhibitory effect on the cell function that is observed when the cytoplasm of said cell is placed into contact with at least one product selected from the group consisting of the "deleted" polypeptides according the invention, the "binding" products according to the invention, the "deleted" polynucleotides according to the invention, the "deleted" transfection vectors according to the invention, the "deleted" adeno-associated virus of the invention, the "deleted" cells according to the invention, under conditions appropriate to cell membrane trafficking,
- the identification of an agent that is capable under physiological conditions to lyse a "binding" product of the invention which is capable of inhibiting at least one of the biological properties a Nter polypeptide can show, as above-defined,
- the identification of an agent that is capable under physiological conditions of stimulating the formation of complexes between compounds which comprise at least one Nter polypeptide of the invention, or of stabilizing such complexes, or of preventing the disruption of such complexes.

For the identification methods of the invention, any appropriate cell is convenient. It notably includes human and animal, normal and pathological cells, neuronal cells, tumor cells.
Said identification may be performed by any means the skilled person find appropriate. This namely includes the screening of chemical and/or biological libraries for an agent having the desired capacity. Screening in function of the capacity of the test agent to bind onto a t-SNARE (such as SNAP25, SNAP23, syntaxin1 and/or syntaxin3 for TI-VAMP) may involve the association of the test products with a tag such as GST, GFP the immobilization of at least one appropriate t-SNARE on a solid support such as a membrane, and the detection by antibodies of those test products which are retained onto said solid support (see *e.g.* overlay assay in the above example 1). Said identification may also be performed by ELISA techniques.

The present invention also encompasses any pharmaceutical agent such as obtained by a method of identification according to the invention. These agents are regulatory agents : the ones obtained by the first method are stimulation agents, the ones obtained by the latter method are inhibition agents.
A further aspect of industrial interest relates to the use of at least one product selected from the group consisting of the Nter polypeptides according to the invention, the Nter polynucleotides according to the invention, the Nter transfection vectors according to the invention, the Nter Adeno-associated virus of the invention, the Nter cells according to the invention, the inhibition pharmaceutical agents according to the invention, for the production of a drug intended for at least one effect selected from the group consisting of inhibiting a cell membrane traffic, inhibiting a vesicular transport, inhibiting a function involving at least one TeNT-resistant pathway, inhibiting a function involving a TeNT-insensitive VAMP such as TI-VAMP, inhibiting the formation of complexes involving at least one TeNT-insensitive VAMP such as TI-VAMP and at least one t-SNARE (such as SNAP25, SNAP23, syntaxin 1, syntaxin 3, for TI-VAMP), inhibiting a cell motility such as the motility of cells susceptible of forming metastasis. The invention notably encompasses an anti-tumor medicament comprising at least one of these products as an active principle. The polynucleotides which are the anti-sense of the "deleted" polynucleotides of the invention may also be used as active principle in such a drug. Such anti-sense polynucleotides, and such a pharmaceutical composition or drug are therefore also encompassed by the present application.

The present invention further relates to the use of at least one product selected from the group consisting of the "deleted" polypeptides according to the invention, the "binding" products according to the invention, the "deleted" polynucleotides according to the invention, the "deleted" transfection vectors according to the invention, the "deleted" adeno-associated virus of the invention, the "deleted" cells according to the invention, the stimulation pharmaceutical agents according to the invention, for the production of a drug intended for at least one effect selected from the group consisting of stimulating a cell membrane traffic, stimulating a vesicular transport, stimulating a function involving at least one TeNT-resistant pathway, stimulating a function involving a TeNT-insensitive VAMP such as TI-VAMP, stimulating the formation of complexes involving at least one TeNT-insensitive VAMP such as TI-VAMP and at least one t-SNARE (such as, for the TI-VAMP : SNAP25, SNAP23, syntaxin 1, syntaxin 3), stimulating axonal and/or dendritic outgrowth, stimuling, neuronal maturation, stimulating neuronal differentiation, preventing or decreasing neuronal, axonal, or dendritic apoptosis, stimulating memory and/or learning capacity, curing and/or palliating and/or preventing spinal cord trauma, curing and/or palliating and/or preventing neuro-degenerative disorders, curing and/or palliating and/or preventing sclerosis. The invention encompasses such a medicament intended for stimulating memory and/or learning capacity, curing and/or palliating and/or preventing spinal cord trauma, curing and/or palliating and/or preventing neuro-degenerative disorders, curing and/or palliating and/or preventing sclerosis, which comprises at least one of these products. The polynucleotides which are the anti-sense of the Nter polynucleotides of the invention may also be used as active principle in such a drug. Such anti-sense polynucleotides, and such a pharmaceutical composition or drug are therefore also encompassed by the present application.

Another aspect of the invention that is of industrial interest relates to a method for diagnosing an undesired state, and/or for assessing the efficiency of a medical treatment, and/or for assessing the evolution of an undesired state, characterized in that it comprises at least one step selected from the group consisting of:
- detecting, in a biological sample, that a product selected from the group consisting of the "deleted" polypeptides according to the invention, the "binding" products according to the invention, the "deleted" polynucleotides according to the invention, the "deleted" transfection vectors according to the invention, the "deleted" adeno-associated virus of the invention, the "deleted" cells according to the invention is present in a quantity or concentration significantly different from the standard level of quantity or concentration (this step will be referred to as step a.), or
- detecting in a biological sample that the expression level of a Nter polypeptide according to the invention is significantly different from the standard expression level (step b.), or
- detecting in a biological sample that the quantity or concentration of complexes involving compounds which comprise at least one Nter polypeptide according to claim 1, is significantly different from the standard level (step c.). Said biological sample may notably be a peripheral blood, tissue or biological liquid sample. It remarkably may correspond to a neuronal cell sample, or to a tumor cell sample.
Said undesired state may correspond to any state selected from the group consisting of the states involving the disregulation of a cell membrane traffic, of a cell TeNT-resistant pathway, of a cell function involving the activity of a TeNT-insensitive VAMP such as TI-VAMP, of the formation of complexes between a TeNT-insensitive VAMP such as TI-VAMP and a t-SNARE (SNAP 25, SNAP 23, syntaxin1, syntaxin3 are t-SNARE for TI-VAMP), of a fusion or an exocytic event. It remarkably may correspond to a state involving the disregulation of the differentiation and/or of the maturation of neuronal cells (disregulation of neurite outgrowth) : this is for example the case of spiral cord trauma, and also of neuro-degenerative disorders, and of certain sclerosis. Said undesired state may also correspond to a state involving the disregulation of the motility of the cells, as this is in particularly the case for tumor cells disseminating so as to form metastasis. Standard is herein meant as the average value observed in healthy patients.
When the quantity/concentration level detected according to the above-defined step a. is significantly superior to the standard level, and/or when the expression level detected in the above-defined step b. is significantly inferior to the standard expression level, and/or when the quantity (or concentration) of complexes as defined in the above step c. is higher than the standard quantity (or concentration), the state concerned is disregulated in the sense of an excess of stimulation (superior level in step a. and/or step c.) or of a lack of inhibition (inferior level in step b.). This may be the case *e.g.* of proliferating and disseminating tumor cells.
When the quantity/concentration level detected according to the above-defined step a. is significantly inferior to the standard level, and/or when the expression level detected in the above-defined step b. is significantly superior to the standard expression level, and/or when the quantity (or concentration) of complexes as defined the above step c. is lower than the standard quantity (or concentration), the state concerned is disregulated in the sense of a lack of stimulation (inferior level in step a. and/or step c.) or of an excess of inhibition (superior level in step b.). This may be the case *e.g.* of non- or insufficiently maturing and/or differentiating neuronal cells.

The invention also encompasses the kits for implementing this method of the invention. Such kits may comprise at least one of said product of the invention, possibly together with appropriate visualization agent such as GFP or GST tag.

The present invention further relates to a method for identifying a compound capable of acting as a biological effector of a TeNT-insensitive VAMP such as TI-VAMP, characterized in that it comprises at least one step selected from the group consisting of:
- the detection of a compound which is capable under physiological conditions of binding to a Nter polypeptide according to the invention,
- the detection of a compound which is capable under physiological conditions of diminishing the inhibition effect that is observed when the cytoplasm of a cell expressing said TeNT-insensitive VAMP is placed into contact with at least one product selected from the group consisting of the Nter polypeptides according to the invention, the Nter polynucleotides according the invention, the Nter transfection vectors according to the invention, the Nter Adeno-associated virus of the invention, the Nter cells according to the invention, under conditions appropriate to cell membrane trafficking,
- the detection of a compound which is capable under physiological conditions of exerting an additional or synergic effect on the cell function that is observed when the cytoplasm of a cell expressing a TeNT-insensitive VAMP such as TI-VAMP is placed into contact with at least one product selected from the group consisting of the "deleted" polypeptides according to the invention, the "binding" products according to the invention, the "deleted" polynucleotides according to the invention, the "deleted" transfection vectors according to the invention, the "deleted" adeno-associated virus of the invention, the "deleted" cells according to the invention, under conditions appropriate to cell membrane trafficking.

Any cell may be appropriate. It remarkably includes human or animal neuronal and tumor cells. Said detection step may be performed according to the common knowledge of the persons skilled in the identification of a compound having the desired capacity. It namely includes screening of chemical and/or biological libraries.
An effector compound such as identified by the method of the invention is of particular use for modulating the activity of its TeNT-insensitive VAMP receptor. It may e.g. be used as a co-agent in the applications described herein.

Other and further characteristics, advantages, and variant embodiments of the invention can be found by the skilled persons from the below-given examples. These examples are given for illustration purposes only, they do not limit the scope of the invention. In particular, while these examples illustrate the invention for the human normal TeNT-insensitive VAMP TI-VAMP, the skilled person can proceed similarly with other TeNT-resistant VAMP, and adjust, when appropriate, the operating conditions.

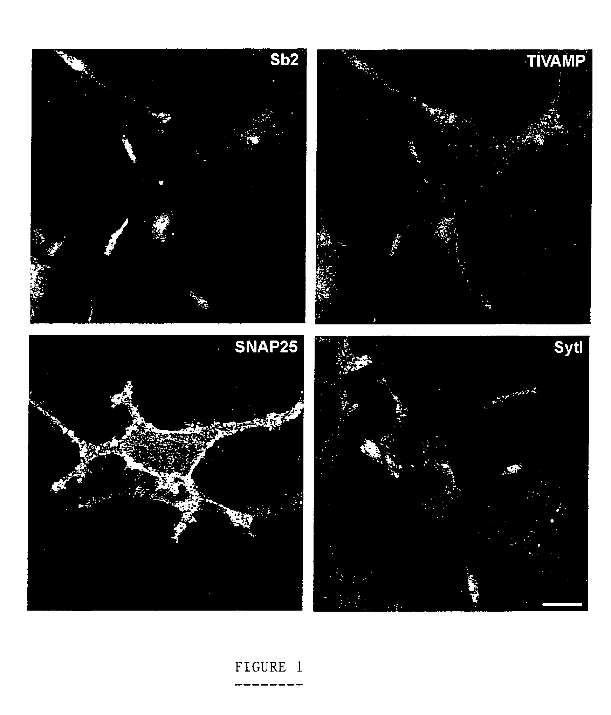

**Figure 1** illustrates the localisation of membrane markers in horizontal confocal sections of staurosporine-differentiated PC12 cells.
PC12 cells were treated with 100nM staurosporine for 24 hours, fixed and processed for immunofluorescence with anti-synaptobrevin 2 (Sb2) and anti-TI-VAMP (TIVAMP), anti-SNAP25 (SNAP25) or anti-synaptotagmin I (Syt I) antibodies. The cells were then observed by confocal microscopy. Note the lack of co-localisation of synaptobrevin 2 and TI-VAMP, the restricted localisation of SNAP25 at the plasma membrane and the concentration of synaptotagmin I at the tip of neurites. Bar: 5µm.

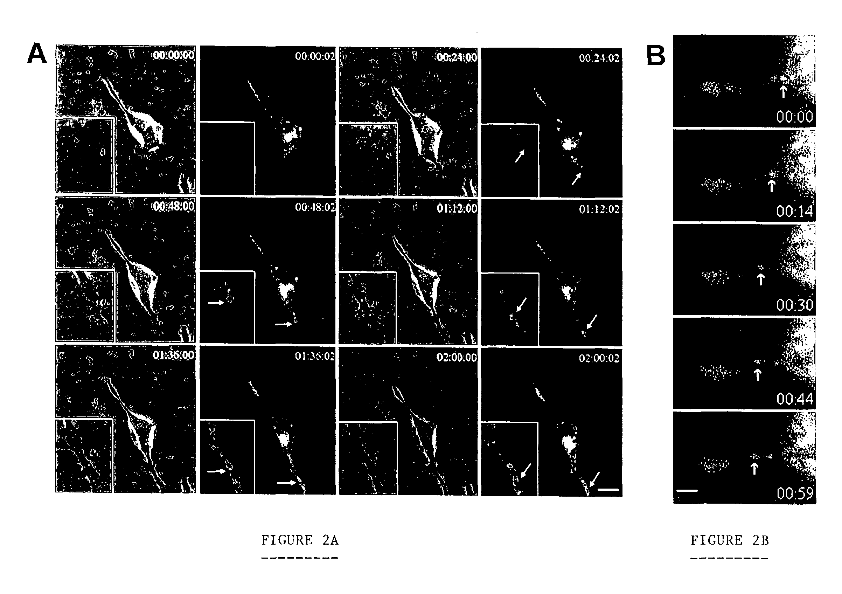

**Figures 2** illustrate the dynamics of GFP-TIVAMP-vesicles : PC12 cells transfected with GFP-TIVAMP were treated with staurosporine for 5 hours and observed under time-lapsed videomicroscopy in the presence of staurosporine.
**Figure 2A** illustrates that the GFP-TIVAMP vesicles accompany the growth of neurites. Transmission and fluorescent light images were recorded every 2 min over a period of 8 hours. Images recorded every 24 min through the middle period of the whole recording are shown. The inset shows a higher magnification of a growing neurite. Arrows indicate regions of this neurite where GFP-TIVAMP concentrates. Bar: 5µm.
**Figure 2B** illustrates the GFP-TIVAMP vesicle dynamics in neurites. Fluorescent light images were recorded every 15s over a period of 30 min (bottom right number: time in s). Images recorded during a lmin period of the recording are shown. The arrow indicates a GFP-TIVAMP vesicles which is moving anterogradly. Bar: 1µm.

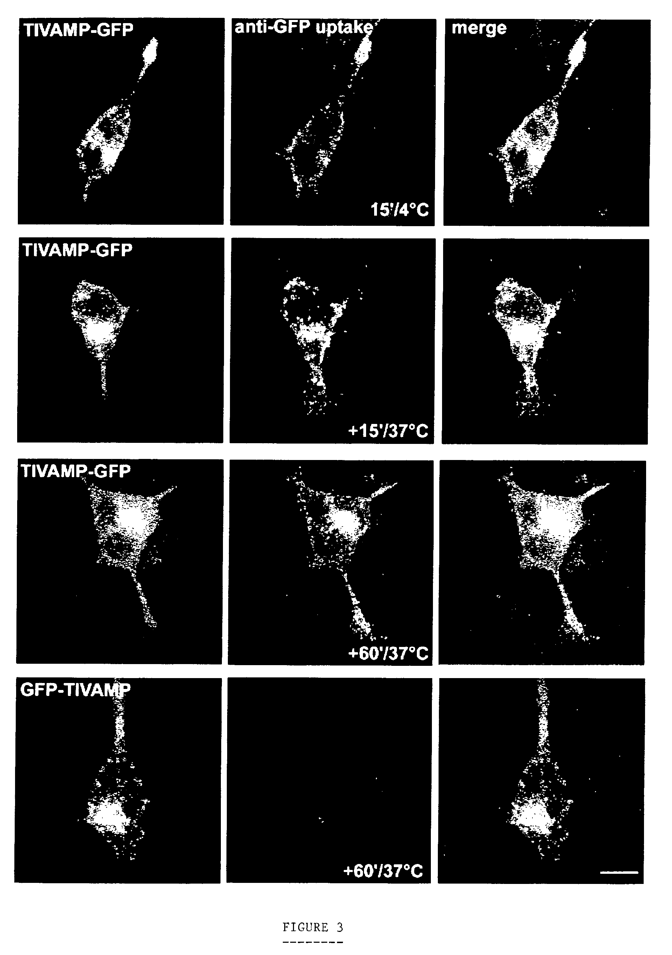

**Figure 3** illustrates TI-VAMP recycles at the neuritic plasma membrane. PC12 cells transfected with TIVAMP-GFP or GFP-TIVAMP and treated with staurosporine for 20 hours were placed on ice, incubated with monoclonal antibody anti-GFP (5µg/ml) for 15 min and directly fixed (15'/4°C) or further incubated at 37°C for 15 min (+15'/37°C) or 60 min (+60'/37°C) before fixation. Note the dense labelling of the neuritic plasma membrane in the 15'/4°C and +15'/37°C conditions. Full loading of the GFP-TIVAMP compartment is reached in the +60'/37°C condition. Bar: 5µm.

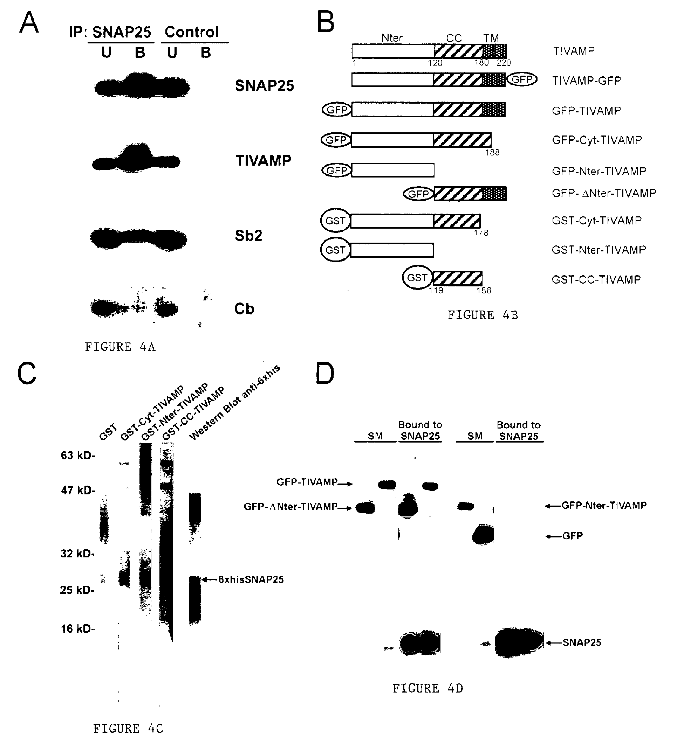

**Figures 4** illustrate the biochemical properties of the TI-VAMP/SNAP25 complex.
**Figure 4A** illustrates that TI-VAMP forms a complex with SNAP25 in Triton X100 extract of rat brain. Immunoprecipitation with anti-SNAP25 antibodies was performed from Triton X100 soluble extract of rat brain as described in the Materials and Methods section of example 1 and immunoprecipitated proteins were detected by western blot analysis with the indicated antibodies (synaptobrevin 2, Sb2; cellubrevin, Cb; U, unbound, B ; bound to anti-SNAP25 immunobeads). The bound fraction corresponded to a 65-fold enrichment compared to unbound. The SNAP25/TI-VAMP complex seemed more abundant than the SNAP25/synaptobrevin 2 one but this may only reflect a lower expression level of TI-VAMP compared to synaptobrevin 2 in the adult brain. Note that cellubrevin did not co-immunoprecipitate with SNAP25.
**Figure 4B** illustrates the structure of TI-VAMP and TIVAMP-derived constructs. TI-VAMP is composed of three domains: the Nter domain (aminoacids 1 to 120), the coiled-coiled domain, also called R-SNARE motif, (CC, amino acids 121 to 180), and one comprising the transmembrane domain and a short luminal domain (TM, amino acids 181 to 220). These domains were tagged with GFP and GST as depicted.
**Figure 4C** illustrates that the Nter domain of TI-VAMP inhibits binding of TI-VAMP to SNAP25.
The binding of GST, GST-Cyt-TIVAMP, GST-Nter-TIVAMP, or GST-CC-TIVAMP was measured by overlay over immobilized 6xhis-SNAP25 (indicated by the arrow). GST-CC-TIVAMP bound efficiently to immobilized 6xhis-SNAP25. Little binding of GST-Cyt-TIVAMP and none of GST and GST-Nter-TIVAMP were observed. As positive control, a strip was revealed with anti-6xhistidine antibodies.
**Figure 4D** illustrates that the TI-VAMP mutant lacking the Nter domain co-immunoprecipitates with SNAP25 more efficiently than full-length TI-VAMP. HeLa cells co-transfected with SNAP25 plus GFP-ΔNter-TIVAMP, GFP-TIVAMP, GFP-Nter-TIVAMP or GFP were lysed and subjected to immunoprecipitation with mouse monoclonal anti-SNAP25 antibodies as described in the Materials and Methods section of example 1. The immuno-precipitated proteins were then detected by western blot with anti-GFP or anti-SNAP25 rabbit polyclonal antibodies. The bound fraction corresponded to a 100-fold enrichment compared to the starting material (SM) in the case of the GFP blot and to a 10-fold enrichment in the case of the SNAP25 blot. Note that neither GFP-Nter-TIVAMP nor GFP co-immunoprecipitated with SNAP25.

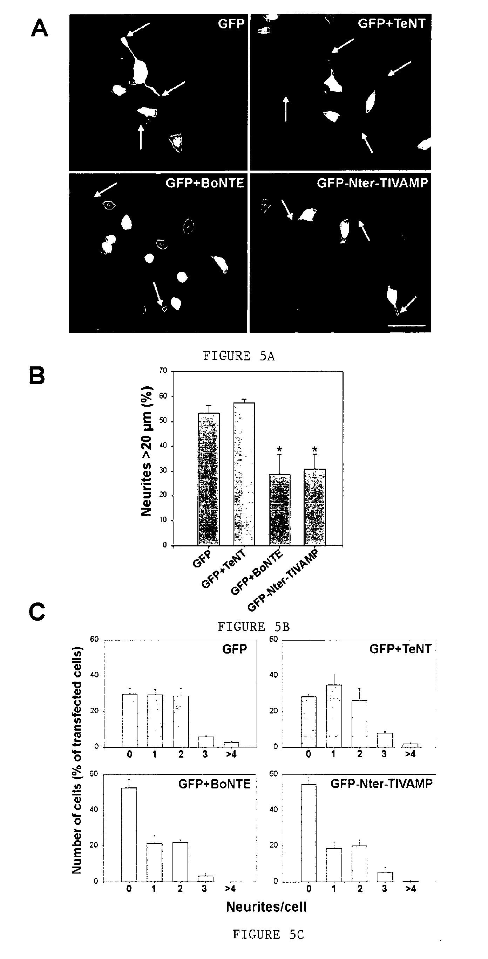

**Figures 5** illustrate the expression of the Nter domain of TI-VAMP inhibits neurite outgrowth.
**Figure 5A** illustrates the effect of GFP, GFP plus TeNT, GFP plus BoNT E or GFP-Nter-TIVAMP on neurite outgrowth. PC12 cells transfected with the indicated constructions and treated with staurosporine were fixed and direct fluorescence images were recorded. Representative fields of the distinct phenotypes found are shown. Note the long neurites displayed both by the GFP and the GFP+TeNT-transfected cells compared with the shorter ones displayed by the GFP+BoNTE and the GFP-Nter-TIVAMP-transfected cells (arrowheads). Bar: 25µm
**Figure 5B** illustrates that GFP-Nter -TIVAMP and BoNT E inhibit neurite length. Percentage of neurites longer than 20µm. A minimun of 50 transfected cells of each type were recorded in blind, and the length of all their neurites was measured. The mean values (+/- SE) of percentage of neurites longer than 20µm from three independent experiments are shown. ^{*}p<0.03 (Student's t-test). Note the lack of effect of TeNT and that BoNT E and GFP-Nter-TIVAMP had a similar inhibitory effect on neurite length.
**Figure 5C** illustrates the number of neurites per cell. The same randomly chosen transfected cells were use to quantify the number of neurites per cell. Shown is the number of cells, expressed as the percentage of transfected cells, displaying 1, 2, 3 or >4 neurites. The mean values (+/- SE) of three independent experiments are shown. Note the lack of effect of TeNT and that both BoNT E and GFP-Nter-TIVAMP enhanced the % of cells without neurites.

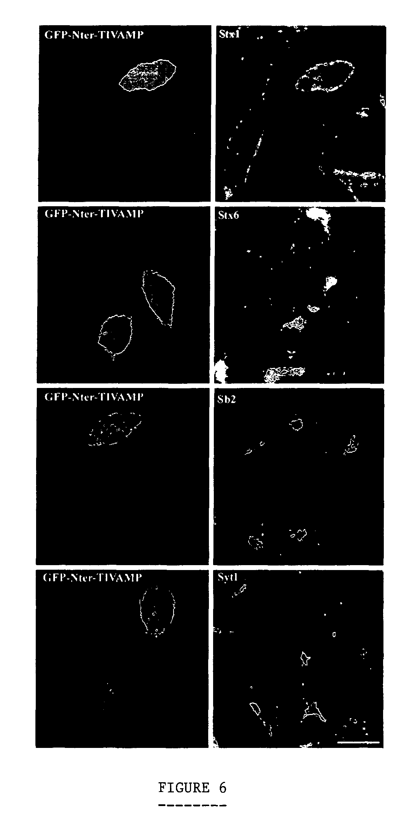

**Figure 6** illustrates the morphology of GFP-Nter-TIVAMP expressing cells. PC12 cells transfected with GFP-Nter-TIVAMP and treated with staurosporine as in figure 5 were fixed, processed for double fluorescence by combining direct GFP fluorescence detection with indirect imunofluorescence detection using the indicated antibodies. Representative GFP-Nter-TIVAMP transfected cells without or with short neurite(s) are shown in horizontal confocal sections. Syntaxin (Stx) 1 and 6 and synaptobrevin 2 (Sb2) have a localisation similar in untransfected as in GFP-Nter-TIVAMP expressing cells. Synaptotagmin I immunoreactivity was weaker in GFP-Nter-TIVAMP transfected cells than in untransfected cells. Bar: 10µm.

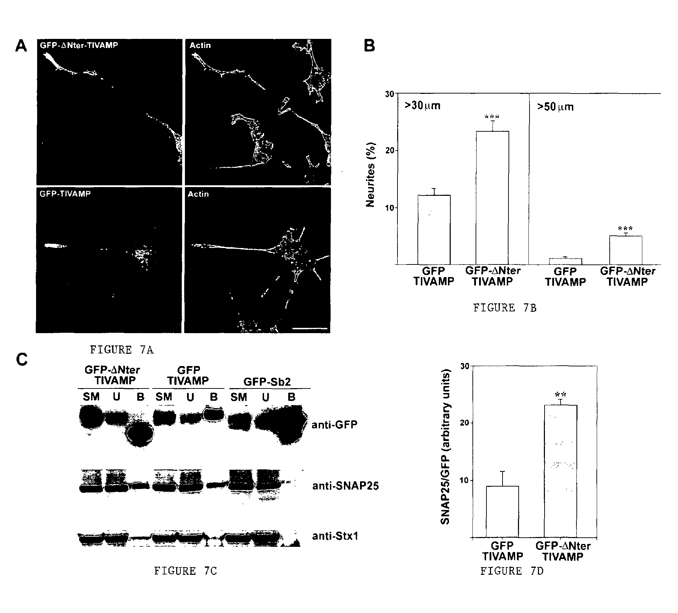

**Figures 7** illustrate that the expression of a "deleted" polypeptide of the invention (TI-VAMP lacking the Nterminal domain) enhances neurite outgrowth.
**Figure 7A** illustrates the morphology of PC12 cells transfected with GFP-TIVAMP or GFP-ΔNter-TI-VAMP. The cells were transfected, treated with staurosporine as in figure 5, fixed/permeabilized and processed for double fluorescence by combining direct GFP fluorescence detection with indirect imunofluorescence detection using Texas Red-phalloidin to visualise the actin filaments. Note the occurrence of numerous filopodia in the neuritic tip of the GFP-ΔNter-TI-VAMP transfected cell. Bar: 10µm
**Figure 7B** illustrates the GFP-ΔNter-TIVAMP increases neurite length. A minimun of 100 transfected cells of each type were recorded in blind, and the length of all their neurites was measured. The mean values (+/- SE) of the percentage of neurites longer than 30µm or 50µm from three independent experiments is shown. ^{***} indicates p< 0.001 (Student's t-test).
**Figure 7C** illustrates the GFP-ΔNter-TIVAMP enhances formation of SNARE complexes. A Triton X-100 soluble extract was prepared from PC12 cells transfected with GFP-TIVAMP, GFP-ΔNter-TIVAMP or GFP-Sb2 and subjected to overnight immunoprecipitation with monoclonal anti-GFP antibodies. Immunoprecipitated proteins were resolved in SDS-PAGE followed by western blot analysis with the indicated antibodies. Note the increased co-immunoprecipitation of endogenous SNAP25 with GFP-ΔNter-TIVAMP compared with GFP-TIVAMP. The histogram in the right side shows the quantification of the amount of endogenous SNAP25 immunoprecipitated normalise to the amount of GFP-fusion protein immunoprecipitated from two independent experiments. **p<0.01 (Student's t-test).

**Figure 8** illustrates the complete human TI-VAMP amino acid (SEQ ID N°6) and polypeptide (SEQ ID N°5) sequences.

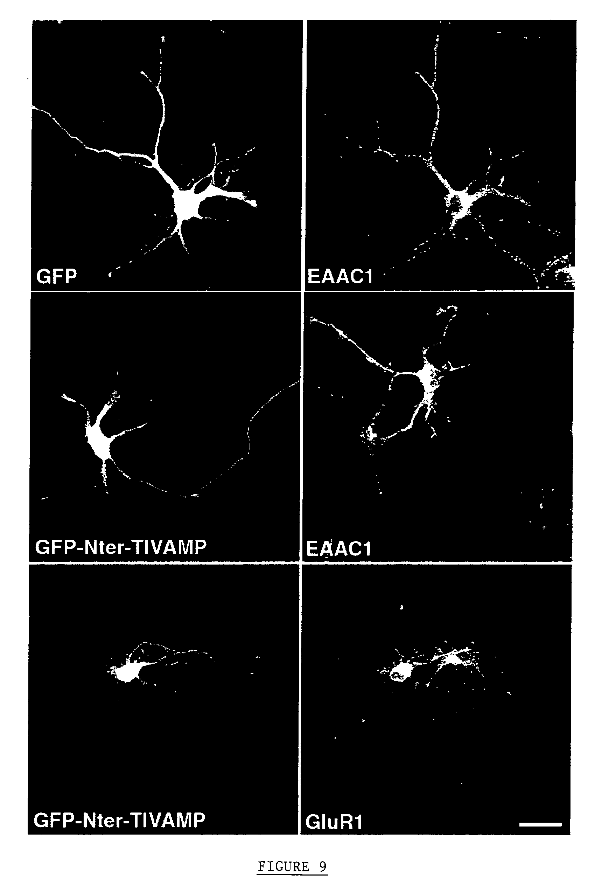

**Figure 9** illustrates that the expression of the amino-terminal domain of TIVAMP specifically affects the distribution of dendritic markers in hippocampal neurons. 4 days old hippocampal neurons from embryonic E18 rats were transfected with GFP or GFP-Nter-TIVAMP and after24 hours fixed and stained for the indicated proteins. Note the expected dendritic localization of EAAC1 in control neurons and in neurons transfected with GFP (right upper panel and nontransfected cell in the right middle panel) compared to its general lower expression and specifically its absence from the dendrites in cells expressing Nter-TIVAMP (transfected cell in the right middle panel). By contrast, the level of expression and the localization of GluR1 were not affected by expression of GFP-Nter-TIVAMP (compare the two cells in the right lower panel). Bar, 21 µm.

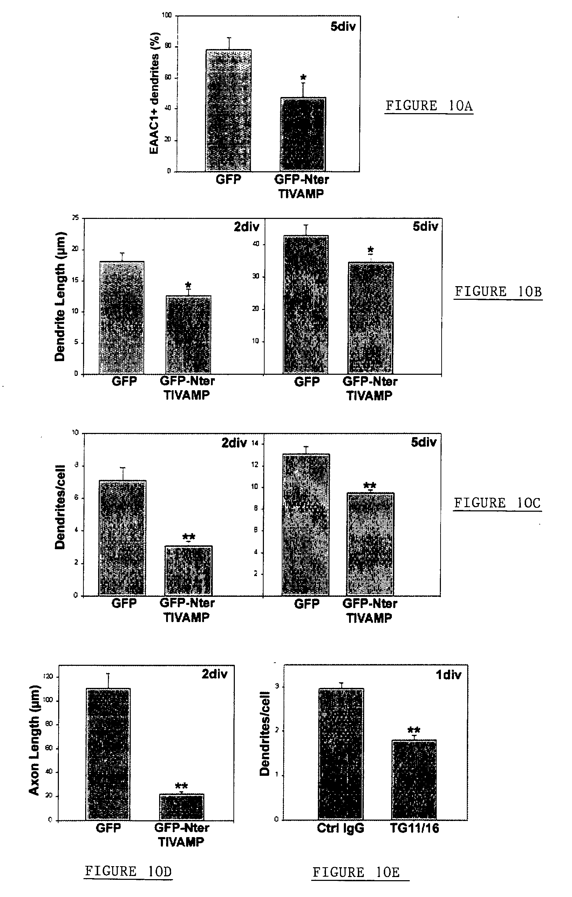

**Figures 10A, 10B, 10C, 10D, 10E** illustrate that the expression of the amino-terminal domain of TIVAMP inhibits axonal and dendritic growth.
**Figure 10A:** Hippocampal neurons from embryonic E18 rats were transfected after 1 div (div = days in vitro) with GFP or GFP-Nter-TIVAMP and the axonal length measured 24h later; shown are the mean values (+/- SEM) of between 40 and 60 analysed cells
**Figure 10B:** Cells transfected as in panel A after 1 div or 4 div were recorded 24 hours later and the dendritic length was measured.
**Figure 10C:** Cells transfected as in figure 10A after 1 div or 4 div were recorded 24 hours post-transfection and the number of dendrites on each cell was counted.
**Figure 10D:** Cells transfected as in figure 10A after 4 div were stained 24 hours later for EAAC1; shown is the mean values (+/- SEM) of percentage of GFP or Nter-TIVAMP positive dendrites labeled also for EAAC1.
**Figure 10E:** Hippocampal neurons from embryonic E18 rats were micro-injected 4 hours after plating with control rabbit IgGs or with affinity-purified anti-TIVAMP rabbit polyclonal antibody, TG11/16. After 20 hours cells were fixed, and the number of dendrites on each injected cell was measured ; shown are the mean values (+/- SEM) of a minimum of 140 recorded cells. **, p<0.006; ^{*}, p<0.06.

**Figures 11A, 11B, 11C, 11D** illustrate that the expression of the amino-terminal domain of TIVAMP induces apoptosis.
**Figure 11A:** Cortical-striatal neurons from intact embryonic brains were electroporated with the indicated constructs and cultured for 24h in the absence (left panels) or presence (right panels) of the caspase inhibitor zVAD. Observe the increase in the number of transfected cells in zVAD-treated Nter-TIVAMP-electroporated cells compared to non treated cells. In the case of GFP-electroporated cells there is no difference between zVAD-treated or non treated cells. Bar, 100 µm.
**Figure 11B:** Quantification of the apoptotic effect of the amino-terminal domain of TIVAMP in cells treated as in figure 11A; shown are the mean values (+/- SEM) of the number of positive cells on each coverslip.

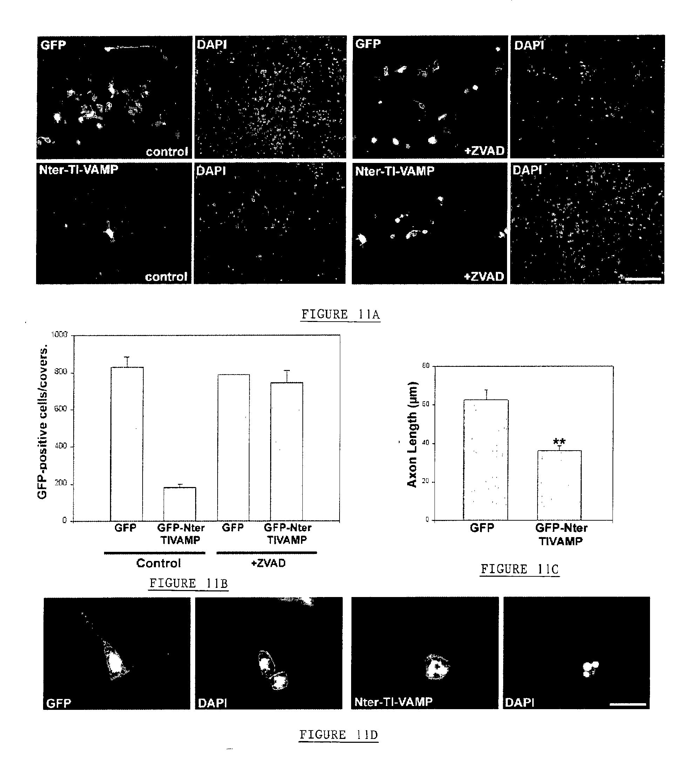

**Figure 11C:** Quantification of the effect in axonal length of the expression of the amino-terminal domain of TIVAMP in cells treated as in figure 11A Shown are the mean values (+/- SEM) of a minimum of 40 cells.
**Figure 11D:** Neurons infected with Aav carrying GFP or Aav carrying GFP-Nter-TIVAMP fixed 3 days after infection. A representative cell of each type is shown. Note that the cell expressing GFP displays neurites and a normal nucleus compared to a noninfected cell, while the cell expressing Nter-TIVAMP is round, with no neurites and presents a typical apoptotic nucleus as seen with DAPI staining. Bar, 20 µm.

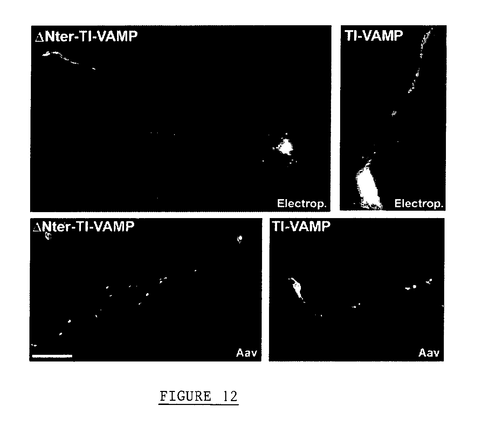

**Figure 12** illustrates that the morphology of neurons expressing TIVAMP or △Nter-TIVAMP. Intact brains from embryonic E13 mice (upper panels) or cortica-striatal neurons from embryonic E16 rats (lower panels) were electroporated or infected with the indicated Aavs, respectively. Cells in primary culture were fixed after 2div (electroporation) or 3div (Aavs). Note the punctuate distribution in the cell body and along the axon of both full-length GFP-TIVAMP and GFP-△Nter-TIVAMP and the fact that GFP-ΔNter-TIVAMP expressing cells present longer axons than cells expressing GFP-TIVAMP. Bar, 20 µm (upper panels); 60 µm (lower panels).

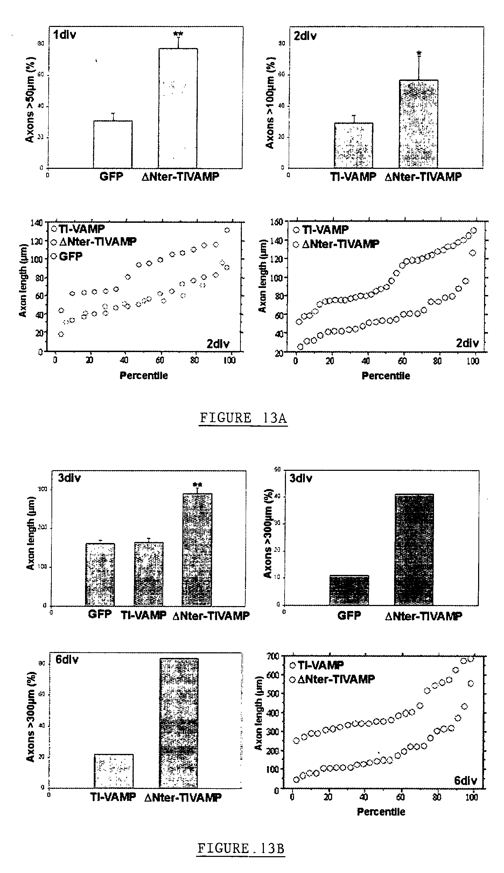

**Figures 13A, 13B** illustrate that the expression of ΔNter-TIVAMP activates axonal growth.
**Figure 13A:** Quantification of the effect in axonal growth of the expression of △Nter-TIVAMP in electroporated neurons. Neurons expressing GFP, GFP-TIVAMP, or GFP-△Nter-TIVAMP were fixed after the indicated times, and the length of their axons was measured. In the upper panels are shown the mean values (+/- SEM) of percentage of axons longer than 50µm or 100 µm from three independent experiments; the lower panels show two representative experiments.
**Figure 13B:** Quantification of the effect on axonal growth of the expression of ΔNter-TIVAMP in Aav-infected neurons. Neurons expressing the indicated constructs were fixed after 3 or 6 div and their axonal length was measured; each panel shows a representative experiment. ^{**}, p<0.001; ^{*}, p<0.005.

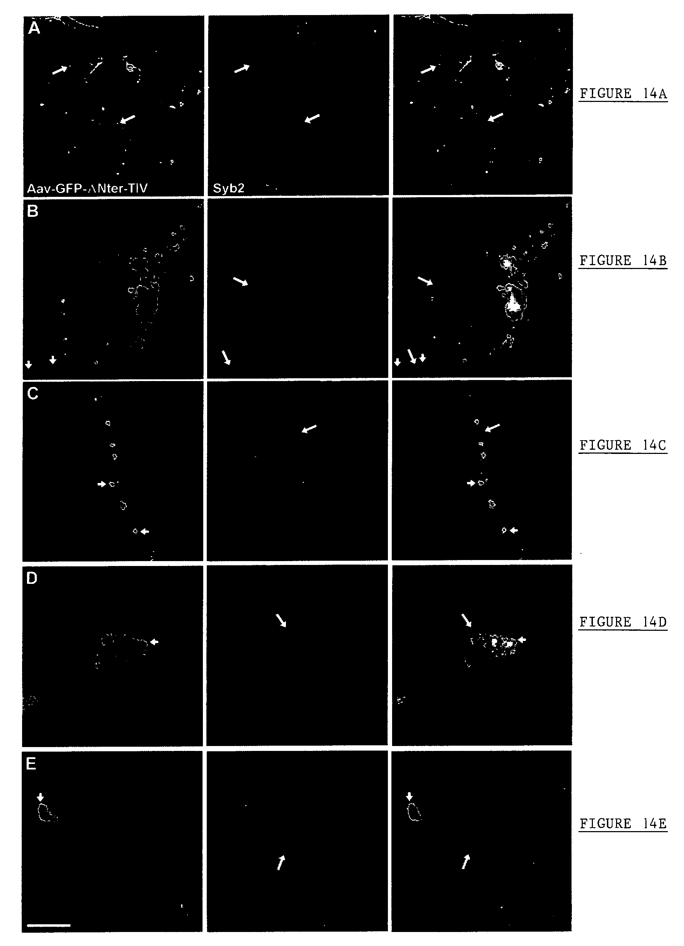

**Figures 14A, 14B, 14C, 14D, 14E** illustrate that GFP-ΔNter-TIVAMP does not colocalize with synaptobrevin 2. Rat embryonic neurons were infected with Aav carrying GFP-ΔNter-TIVAMP. After 6 div, the cells were fixed and permeabilized, incubated with a polyclonal antibody anti-GFP and with a monoclonal antibody anti-synaptobrevin 2, and observed by confocal microscopy. Low magnification images are shown in **Figure 14A.** In all the other panels high magnification images of a cell body **(Figure 14B),** an axon **(Figure 14C),** a varicosity **(Figure 14D)** and a growth cone **(Figure 14E),** respectively are shown. GFP-ΔNter-TIVAMP (small arrows) does not co-localize with endogenous synaptobrevin 2 (big arrows in Figures 14B, 14C, 14D and 14E) in any of the different neuronal domains. A significant amount of GFP-ΔNter-TIVAMP was detected at the leading edge of the growth cone, in a region devoid of synaptobrevin 2. Bars: Figure 14A, 90 µm ; Figures 14B, 14C, 14E, 4.6 µm ; Figure 14D, 3 µm.

### EXAMPLE 1:

### Materials and Methods

**Antibodies and clones:** Rabbit serums (TG11 and TG16) directed against TIVAMP were purified by affinity chromatography in a column loaded with a GST-fusion protein of the coiled-coil domain of TI-VAMP (see below).

Mouse monoclonal antibodies directed against synaptobrevin 2 (clone 69.1, available from Max Planck Institute, Goettingen, FRG), SNAP25 (clone 20, Transduction Labs., San Diego, CA), GFP (clone 7.1 and 13.1, Boehringer, Mannheim, FRG), syntaxin 6 (clone 30, Transduction Labs, San Diego, CA), syntaxin 1 (HPC-1, available from Yale University, New Haven, CT), glutathione S-transferase (generous gift from J.-L. Theillaud, Institut Curie, Paris, France), TeNT light chain (TeNT-LC) (generous gift from H. Niemann, Hannover Medical School, Hannover, FRG), rabbit polyclonal antibodies against the ectoplasmic domain of synaptotagmin I (8907, available from Yale University, New Haven, CT), SNAP25 (MC9, available from Yale University, New Haven, CT), GFP (Boehringer, Mannheim, FRG) and histidine (Santa Cruz Biotechnology Inc., Santa Cruz, CA) have been described previously. Affinity-purified Cy2 and Texas Red-coupled goat anti-mouse and anti-rabbit immunoglobulins were purchased from Jackson ImmunoResearch (West Grove, PA). Rhodamine-coupled phalloidin was from SIGMA (Saint-Louis, MO). Alkaline Phosphatase coupled-sheep anti-mouse were from Promega (Madison, WI). The cDNAs of human TI-VAMP and cellubrevin were previously described. The cDNA sources were : rat synaptobrevin 2 (R. Scheller, Stanford University, Stanford, CA), for rat SNAP25A (R. Jahn, Max Planck Institute, Goettingen, FRG), for TeNT-LC and BoNT-LC (H. Niemann, Hannover Medical School, Hannover, FRG) and for ratiometric pHLuorin (G. Miesenbock, Sloan Kettering Memorial Hospital, New York, NY).

**Cell culture**: PC12 cells were cultured in RPMI supplemented with 10% horse serum (HS) and 5% foetal calf serum (FCS) under standard conditions for PC12 cells. Cells were plated either on collagen-coated plastic dishes or on poly(L)lysine plus collagen-coated glass coverslips. HeLa cells were cultured in DMEM supplemented with 10% FCS.

**DNA constructions:** For production of N-terminal-GFP-fusion proteins the distinct cDNAs were cloned into the pEGFP-C3 vector (Clontech, Palo Alto, CA). The same empty vector was also used as a control in some of the neurite outgrowth assays. Full-length TIVAMP (TIVAMP; aa : SEQ ID N°6 ; DNA : SEQ ID N°5), N-terminal domain-TIVAMP (Nter-TIVAMP, from M¹ to N¹²⁰ ; aa : SEQ ID N°2 ; DNA : SEQ ID N°1), cytosolic domain-TIVAMP (Cyt-TIVAMP, from M¹ to K¹⁸⁸ ; aa : SEQ ID N°8 ; DNA : SEQ ID N°7), ΔN-terminal domain-TIVAMP (ΔNter-TIVAMP, from M¹⁰² to the end ; aa : SEQ ID N°4 ; DNA : SEQ ID N°3), and full-length synaptobrevin 2 (Sb2), were obtained by PCR using standard procedures and the following sets of oligonucleotides:
5'-ATGGCGATTCTTTTTGCTGTTGTTGCC-3' (SEQ ID N°9) and 5'CTATTTCTTCACACAGCTTGGCCATGT-3' (SEQ ID N°10) for TI-VAMP; 5'-ATGGCGATTCTTTTTGCTGTTGTTGCC-3' (SEQ ID N°11) and 5'-CTTATTCTCAGAGTGATGCTTCAGCTG-3' (SEQ ID N°12) for Nter-TI-VAMP; 5'-ATGGCGATTCTTTTTGCTGTTGTTGCC-3' (SEQ ID N°13) and 5'-ATCCTACTTGAGGTTCTTCATACACATGGCTC (SEQ ID N°14) for Cyt-TI-VAMP; 5'-ATGAATAGCGAGTTCTCAAGTGTCTTA-3' (SEQ ID N°15) and 5'-CTATTTCTTCACACAGCTTGGCCATGT-3' (SEQ ID N°16) for ΔNter-TI-VAMP; 5'-ATGTCGGCTACCGCTGCCACCGTCCCG-3' (SEQ ID N°17) and 5'-TTAAGAGCTGAAGTAAACTATGATGAT for Sb.2 (SEQ ID N°18). TI-VAMP, Nter-TI-VAMP, Cyt-TI-VAMP and Cyt-Syb2 were cloned in pEGFP-C3 using the KpnI/XbaI sites, while ΔNter-TI-VAMP was cloned in HindIII/XbaI sites.
On figure 8, is shown the complete human TI-VAMP sequences SEQ ID N°5 (DNA) and SEQ ID N°6 (amino acids; from M¹ to K²²⁰).
Nter amino acid sequence corresponds to human TI-VAMP sequence from M¹ to N¹²⁰ (SEQ ID N°2). Nter DNA sequence bears SEQ ID N°1.
ΔNter-TI-VAMP amino acid sequence corresponds to human TI-VAMP sequence from M¹²⁰ to K²²⁰ (SEQ ID N°4). △Nter-TI-VAMP DNA sequence bears SEQ ID N°3.
Cytosolic TI-VAMP amino acid sequence corresponds to human TI-VAMP sequence from M¹ to K¹⁸⁸ (SEQ ID N°8). Cyt-TI-VAMP DNA sequence bears SEQ ID N°7.
The 101 amino acid fragment which lacks from ΔNter-TI-VAMP by comparison with the complete human TI-VAMP sequence, i.e. the M¹-A¹⁰¹ fragment, bears SEQ ID N°20. Its corresponding DNA sequence bears SEQ ID N°19.

For production of the C-terminal-GFP (ratiometic pHLuorin in this case) fusion protein of TIVAMP, TIVAMP cDNA bearing a BamHI site in its 3' was obtained by PCR using the 5'-GGATCCTTTCTTCACACAGCTTGGCCA-3' (SEQ ID N°21) and 5'-CTATTTCTTCACACAGCTTGGCCATGT-3' (SEQ ID N°22) oligonucleotides, and cloned in the pCR3.1-Uni vector (Clontech, Palo Alto, CA). Ratiometric pHLuorin was then cloned in the BamHI/EcoRI sites. Nter-TIVAMP, Cyt-TIVAMP, and coiled-coiled domain of TIVAMP (CC-TIVAMP; from E"9 to K¹⁸⁸) were fused to glutathione S-tranferase (GST) gene by cloning in pGEX4T vector (Pharmacia, Saclay, France).

**Overlay assay:** The corresponding GST fusion proteins and GST alone were produced and purified as previously described. 6xhis-tagged SNAP25A (6xhisSNAP25, bacterial strain is available from G. Schiavo, ICRF, London, UK) was purified.
6xhisSNAP25 was run on SDS-PAGE and western blotted onto Immobilon-P membrane (Millipore, Bedford, MA). The amount of 6xhisSNAP25 corresponds to 1.25µg/mm of membrane. 4mm strips of the membrane were cut and incubated in 150mM NaCl, 5% non fat dry milk, 50mM phosphate pH7.5 buffer for 1 hour at room temperature. The strips were then incubated with 10nM the GST-fusion proteins overnight at 4°C in buffer B (3% BSA, 0.1% Tween 20, 20mM Tris pH: 7.5) containing 1 mM DTT. The strips were rinsed three times in buffer B at room temperature, incubated with anti-GST antibodies in buffer B for 1 hour, rinsed in buffer B three times and incubated with alkaline phosphatase-coupled sheep anti-mouse antibodies. The detection was carried out simultaneously for all the strips, for the same time, using a kit from Promega (Madison, WI).

**Cell transfection:** PC12 or HeLa cells were trypsinized, washed and re-suspended at a density of 7.5-10 x 10⁶ cells/ml in Optimix (Equibio, Boughton Monchelsea, UK). Electroporation was performed with 10µg DNA in a final volume of 0.8ml cell suspension using a Gene Pulser II device (Bio-Rad, Hercules, CA) with one shock at 950µF and 250 V. When GFP was co-transfected with TeNT or BoNTE for monitoring the transfected cells, the plasmid carrying the GFP gene was added at double concentration in order to ensure that all the cells that uptake it do also uptake the plasmid carrying the toxin. Immediately after electroporation, cells were washed with 5ml of complete medium before plating them for immunoprecipitation or immunofluorescence microscopy analysis. Five hours later, the outgrowth medium was removed and replaced with fresh medium containing 100nM staurosporine (SIGMA, St Louis, MO). PC12 and HeLa cells were processed 24 or 48 hours after transfection respectively. For enhanced expression of the exogenous proteins, 5mM sodium butyrate was added in all the cases during the last 6 hours before processing the cells.

**Antibody uptake assay:** PC12 cells processed as indicated above were incubated in the presence of 5µg/ml anti-GFP antibody in culture medium for 15min on ice, 15min on ice then 15min at 37°C, or 15min on ice then 60min at 37°C, 24 hours after transfection with GFP-TIVAMP or TIVAMP-GFP. The cells were then washed twice with culture medium and twice with PBS, fixed with PFA and processed for immunofluorescence.

**Immunocytochemistry:** Cells were fixed with 4% PFA and processed for immunofluorescence. Optical conventional microscopy was performed on a Leica microscope equipped with a MicroMax CCD camera (Princeton Instruments, Princeton, NJ). Confocal laser scanning microscopy was performed using a TCS confocal microscope (Leica, Heidelberg, FRG). Images were assembled without modification using Adobe Photoshop (Adobe Systems, San Jose, CA ).

**Neurite outgrowth assay:** Cells were fixed 24 hours after transfection. Between 20 and 100 randomly chosen fields for each condition were taken with a MicroMax CCD camera (Princeton Instruments, Princeton, NJ), resulting in the analysis of at least 50 transfected cells. A neurite was defined as a thin process longer than 5µm. Using the Metamorph software (Princeton Instruments, Princeton, NJ) two parameters were scored in each case: the number of neurites per cell (from 0 to 4 or more neurites), and the length of each neurite, from the cell body limit until the tip of the process. The obtained data were analysed for their statistical significance with SigmaStat (SPSS Inc., Chicago, IL). All the recordings and the Metamorph analysis were done in blind.

**Videomicroscopy:** Living PC12 cells transfected and treated with staurosporine as described above were placed in complete medium in an appropriate chamber equilibrated at 37°C and 5% CO₂. Cells were monitored with a MicroMax CCD camera (Princeton Instruments, Princeton, NJ) for as much as 9 hours, taking images both through phase contrast and FITC fluorescence every 2 min or every 15 seconds. Images were assembled using Metamorph (Princeton Instruments, Princeton, NJ).

**Immunoprecipitation:** Immunoprecipitation from rat brain was performed using a Triton X-100 soluble membrane fraction prepared as follows: two adult rat brains were homogenized with a glass/teflon homogenizer (9 strokes at 900 rpm) in 25 ml of 0.32M sucrose containing a protease inhibitor cocktail. All the steps were carried out at 4°C. After 10 min centrifugation at 800g the supernatant was centrifuged at 184000g for 1 hour, obtaining a cytosolic and a membrane fraction in the supernatant and the pellet, respectively. The pellet was re-suspended in TSE (50mM Tris pH 8.0, 0.5mM EDTA, 150mM NaCI) containing 1% Triton X-100 for 30 min and finally the insoluble material was removed by centrifugation at 184000g for 1 hour. Immunoprecipitation with anti-SNAP25 antibodies and mouse control IgGs was performed from 2mg of proteins from the soluble extract. Immunoprecipitations from transfected PC12 or Hela cells were performed using a total Triton X-100 soluble fraction prepared as follows: after two washes with cold TSE, cells were lysed for 1 hour under continuous shaking with TSE containing 1% Triton X-100 and protease inhibitors. The supernatant resulting from centrifugation at 20000g for 30 min was used for immunoprecitation. After overnight incubation of the brain and cell extracts with the antibodies, 50 µl of magnetic beads (Dynabeads, Dynal A.S., Oslo, Norway) were added for 2-4 hours. The magnetic beads were washed four times with TSE containing 1%Triton X-100, eluted with gel sample buffer and the eluates were boiled for 5 min and run on SDS-PAGE gels.
ΔNter-TI-VAMP amino acid sequence corresponds to human TI-VAMP sequence from M¹²⁰ to K²²⁰ (SEQ ID N°3). ΔNter-TI-VAMP DNA sequence bears SEQ ID N°4.
Cytosolic TI-VAMP amino acid sequence corresponds to human TI-VAMP sequence from M¹ to K¹⁸⁸ (SEQ ID N°7). Nter DNA sequence bears SEQ ID N°8.
The 101 amino acid fragment which lacks from △Nter-TI-VAMP by comparison with the complete human TI-VAMP sequence, i.e. the M¹-A¹⁰¹ fragment, bears SEQ ID N°19. Its corresponding DNA sequence bears SEQ ID N°20.

### Results

### TI-VAMP dynamics in staurosporine-treated PC12 cells

Differentiation of neurons and nerve growth factor (NGF)-induced neurite outgrowth of PC12 cells take several days. On the contrary, staurosporine, a protein kinase inhibitor, induces maximal neurite outgrowth in 24 hours of treatment in PC12 cells. Our neurite outgrowth assay is based on treating PC12 cells with 100 nM staurosporine for 24 hours. These experimental conditions do not induce apoptosis in PC12 cells. Figure 1 shows that synaptobrevin 2, TI-VAMP, SNAP25 and synaptotagmin I had a normal subcellular localisation in staurosporine-treated PC12 cells. Synaptobrevin 2 concentrated in the perinuclear region and in neuritic tips. TI-VAMP positive vesicles were scattered throughout the cytoplasm and concentrated at the leading edge of extending neurites. Synaptotagmin I appeared almost exclusively in neurites and varicosities and SNAP25 was present throughout the plasma membrane. This pattern of immuno-staining was similar to that observed in NGF-treated PC12 cells, demonstrating the validity of this cellular model to study neurite outgrowth.

We produced TI-VAMP carrying a Green Fluorescent Protein (GFP) tag fused to the N-terminal end (GFP-TIVAMP, Figure 4B). Upon transfection of this construct in PC12 cells, GFP staining was indistinguishable from that of endogenous TI-VAMP (compare figure 2A with figure 1), thus discarding the possibility that fusion of the GFP tag could alter TI-VAMP trafficking. We then observed TI-VAMP dynamics by time lapsed video-microscopy in staurosporine-treated PC12 cells, which had been previously transfected with GFP-TIVAMP (figure 2). Fast growing neurites were recorded every 2 min over periods of 3 to 9 hours, 5 hours after the onset of staurosporine treatment. Figure 2A displays transmission and fluorescent light images recorded every 24 min during 2 hrs 02 min (see also accompanying movie). High magnification view of a neurite growing towards the bottom right of the image is shown in the inset. At each time point, GFP-TIVAMP containing vesicles distributed along this growing process, up to the leading edge of the growth cone (Figure 2A). Most movements of GFP-TIVAMP containing membranes were anterograde (Figure 2B).

We then constructed another form of fluorescent TI-VAMP by introducing a GFP tag at the C-terminus (TIVAMP-GFP, Figure 4B). In this case, the GFP tag is exposed to the extracellular medium following exocytosis of TI-VAMP containing vesicles. TIVAMP-GFP transfected PC12 cells were labelled with monoclonal antibodies directed against GFP while they were placed on ice, before fixation. The labelling was often concentrated at the tip of the growing neurite (Figure 3). When the cells were allowed to internalise the antibody at 37°C, we observed a fast, time-dependent uptake. After 15 min at 37°C, the anti-GFP immuno-reactivity was seen in peripheral structures, very close to the plasma membrane with a low degree of overlap with the green signal emitted by the bulk of TIVAMP-GFP. After 60 min, most of the immuno-reactvity co-localised with TIVAMP-GFP, indicating that the anti-GFP antibody had reached the entire TIVAMP-GFP compartment. We did not detect any plasma membrane labelling nor GFP antibody internalisation in GFP-TIVAMP transfected or untransfected cells (Figure 3) thus demonstrating the lack of capture of the antibody by fluid phase uptake. Altogether, these studies demonstrate that the dynamics of TI-VAMP containing vesicles very closely accompanies the growth of neurites and that the protein recycles at the neuritic plasma membrane.

### The N-terminal domain of TI-VAMP inhibits SNARE complex formation

Because TI-VAMP resists to NT treatment, new experimental approaches had to be developed to study its function in living cells. Toward this goal, we searched for mutated forms of TI-VAMP that would have impaired SNARE complex formation activity. We first identified SNAP25 as a main physiological target SNARE (t-SNARE) of TI-VAMP. SNAP25, a neuronal plasma membrane Q-SNARE, formed abundant SNARE complexes with TI-VAMP as seen by co-immunoprecipitation experiments performed from brain extracts. Cellubrevin, a v-SNARE that is expressed in glial cells but not in neurons, did not associate with SNAP25 thus showing that the SNARE complexes were not formed during solubilisation of brain membranes (figure 4A).

Protein sequence analysis of TI-VAMP shows that the protein has an original N-terminal (Nter) domain of 120 amino acids, located upstream of the coiled-coiled domain (also called R-SNARE motif). This Nter domain includes three regions predicted to be α helical by Hydrophobic Cluster Analysis and Jpred. This is reminiscent of the Nter domain of syntaxin 1, which comprises 3 α helices and inhibits lipid bilayer fusion. The Nter domain of Sso1p, the yeast homologue of syntaxin 1, inhibits the rate of SNARE complex formation. Similar Nter domains are present in the other plasma membrane but not in intracellular syntaxins, indicating that this function may be specific for exocytosis. This led us to prepare the following GST-fusion proteins: full cytoplasmic domain of TI-VAMP (GST-Cyt-TIVAMP), coiled-coiled domain alone (GST-CC-TIVAMP) and Nter domain alone (GST-Nter-TIVAMP) (Figure 4B), and to measure the binding of the corresponding proteins to immobilized 6xhis-SNAP25 in an overlay assay. GST-CC-TIVAMP bound very efficiently immobilized his-SNAP25 whereas GST-Cyt-TIVAMP bound very poorly. As controls, GST alone and GST-Nter-TIVAMP did not bind immobilized his-SNAP25 (Figure 4C). HeLa cells do not express endogenous SNAP25 so, we used them to study the association of SNAP25 with GFP-TIVAMP, GFP-ΔNter-TIVAMP, GFP-Nter-TIVAMP (Figure 4B) or GFP, *in vivo*, following co-transfection. We measured the amount of GFP-tagged proteins co-immunoprecipitating with SNAP25 from Triton X-100 soluble extracts. GFP-ΔNter-TIVAMP formed more abundant SNAP25-containing SNARE complexes than GFP-TIVAMP. As controls, GFP and GFP-Nter-TIVAMP did not bind SNAP25 (figure 4D). Altogether, we propose that the Nter domain exerts an intramolecular inhibition of the SNARE complex formation activity of TI-VAMP's coiled-coiled domain.

### TI-VAMP mediates neurite outgrowth

An assay was set up to measure the effect of transfection of NTs and TI-VAMP mutants on staurosporine-induced neurite outgrowth in PC12 cells. First, we showed that when cells were electroporated with two plasmids, virtually all cells expressed both transgenes. This was demonstrated by transfection with GFP-cellubrevin (GFP-Cb) alone, TeNT alone, or both. Co-transfection of TeNT with GFP-Cb resulted in total proteolysis of GFP-Cb. Second, the activity of transfected TeNT and BoNT E were demonstrated by complete proteolysis of endogenous synaptobrevin 2 and SNAP25 respectively.

In a first set of experiments, PC12 cells were transfected with GFP alone, GFP plus TeNT, GFP plus BoNT E or the Nter domain of TI-VAMP fused to GFP (GFP-Nter-TIVAMP, Figure 4B). The cells were then treated with staurosporine, and fixed after 24hours. Figure 5A shows a representative field observed in each condition. Neurites from cells transfected with GFP or GFP plus TeNT were similar to neurites from untransfected cells. Neurites from cells transfected with GFP plus BoNT E or GFP-Nter-TIVAMP were fewer and shorter. The length of neurites and the number of neurites per cell were measured in each GFP-positive cell, in each condition. GFP plus TeNT had no effect on neurite number and length compared to GFP alone. BoNT E reduced by 45% the number of neurites longer than 20 µm and strongly increased the number of cells without neurites (figure 5B,C). Expression of the Nter domain of TI-VAMP had an effect which was similar to that of BoNT E. GFP-Nter-TIVAMP reduced by 42% the number of neurites longer than 20µm and strongly increased the number of cells without neurites (figure 5B,C). The effects of GFP plus BoNT E and GFP-Nter-TIVAMP were statistically different from GFP alone with p= 0.027 and 0.017 (Student's t-test) respectively. The effects of BoNT E and GFP-Nter-TIVAMP were not additive, indicating that they act on the same exocytotic mechanism. In a different set of experiments, we measured the effect of GFP and GFP-Cyt-TIVAMP. GFP-Cyt-TIVAMP (neurites longer than 20µm: 50.2%+/-0.25) had no effect on neurite length compared to GFP (neurites longer than 20 µm: 50.7%+/-3.5). GFP-Cyt-TIVAMP had no effect on the number of neurites per cell. These results demonstrated that neurite outgrowth in staurosporine-treated cells is insensitive to TeNT but sensitive to BoNT E as in neurons. The fact that GFP-Nter-TIVAMP inhibited neurite outgrowth as strongly as BoNT E suggests that TI-VAMP plays a major role in neurite outgrowth.

We then checked that GFP-Nter-TIVAMP expression did not have a deleterious effect. Figure 6 shows a gallery of double immunofluorescence experiments performed in GFP-Nter-TIVAMP transfected cells. We observed no effect on the localisation of syntaxin 1, a plasma membrane SNARE, syntaxin 6, a Golgi apparatus SNARE (figure 6), and SNAP25 when compared to untransfected or GFP-transfected cells. Synaptobrevin 2 appeared both in the perinuclear region and in the shorter neurites emerging from GFP-Nter-TIVAMP cells (figure 6 and compare to figure 1). These cells showed a lower level of expression of synaptotagmin I. Synaptotagmin I was the vesicular marker which was the most enriched in the tip of the neurites in untransfected cells (Figure 1 and 6) so our result may suggest that synaptotagmin I reached the neuritic tip by a TI-VAMP dependent pathway. These results showed that the Nter domain of TI-VAMP had a specific inhibitory effect on neurite outgrowth.

We then tested the effect of GFP- △Nter-TIVAMP expression and compared it with that of GFP-TIVAMP on neurite outgrowth. We observed the occurrence of unusually long neurites with an increased number of filopodia. Staining of actin filaments with fluorescent phalloidin showed that the neurites of GFP□ΔNter-TIVAMP-transfected cells showed cortical actin localisation similar to GFP-TIVAMP-transfected cells (Figure 7A). The pattern of staining of tubulin, synaptobrevin 2, synaptotagmin 1, SNAP25 and syntaxin 1 was the same in GFP- ΔNter-TIVAMP as in GFP-TIVAMP transfected and in untransfected cells. The effect of GFP- ΔNter-TIVAMP was quantified as in the case of GFP- ΔNter-TIVAMP. GFP- ΔNter-TIVAMP expression doubled the number of neurites longer than 30 µm and multiplied by 5 the number of neurites longer than 50 µm when compared to the expression of GFP-TIVAMP (Figure 7B). GFP-TIVAMP had no effect on neurite length and number per cell compared to GFP alone. We observed no effect of GFP- ΔNter-TIVAMP on the number of neurites per cell. We checked that GFP- ΔNter-TIVAMP formed more abundant SNARE complexes with endogenous SNAP25 by measuring the amount of SNAP25 and syntaxin 1 which was co-immunoprecipitated with GFP-△Nter-TIVAMP, GFP-TIVAMP and GFP-Syb2. GFP- ΔNter-TIVAMP /SNAP25 complex was 2.5 times more abundant than GFP-TIVAMP/SNAP25. Accordingly, GFP-△Nter-TIVAMP co-immunoprecipitated more syntaxin 1 than GFP-TIVAMP (Figure 7C). These results showed that a form of TI-VAMP, which had a higher SNARE complex formation activity strongly, enhanced neurite outgrowth.

### Discussion

Tetanus neurotoxin Insensitive-Vesicle Associated Membrane Protein (TI-VAMP : DNA sequence SEQ ID N°5 ; aa sequence SEQ ID N°6) is a V-SNARE (vesicle-associated soluble N-ethylmaleide-sensitive fusion protein attachment receptor) which is known to be involved in transport to the apical plasma membrane in epithelial cells.
The present invention reports for the first time that a N-terminal fragment of a VAMP such as TI-VAMP can show biological functions *in vivo*.
It is herein demonstrated that a fragment corresponding to the N-terminal domain which precedes the SNARE motif of TI-VAMP plays an inhibitory role on the activity of the VAMP TI-VAMP, that SNAP25 is a target SNARE (t-SNARE) for TI-VAMP in cells such as neuronal cells (i.e. they form complexes in such cells), and that such a N-terminal fragment is capable of inhibiting the association of TI-VAMP with its target SNARE (SNAP25 in neuronal cells, SNAP23 in epithelial cells). The first 120 N-terminal aa fragment corresponds to SEQ ID N°2 (corresponding DNA sequence: SEQ ID N°1); the first 101 ones to SEQ ID N°20 (corresponding DNA sequence: SEQ ID N°19). Such N-terminal fragments are capable of inhibiting the membrane traffic activity of the cells into which they have been transfected : they inhibit their fusion functions, and in a particular aspect, their exocytic functions. Membrane traffic can be envisioned as a succession of vesicle budding, maturation, vectorial transport, tethering, docking, and lipid bilayer fusion events. Vesicular transport to and fusion at the plasma membrane, *i*.*e*. exocytosis, is responsible for the release of soluble compounds such as neurotransmitters in the extracellular medium and for surface expression of plasma membrane proteins and lipids. On a more mechanistic point of view, such Nter fragments are herein shown to be capable of inhibiting the formation of complexes involving VAMP, and notably complexes involving a VAMP such as TI-VAMP and at least one of its t-SNARE (SNAP 25, SNAP23, syntaxin1, syntaxin3). In fact, such N-terminal fragments may inhibit any cell function which involves a tetanus neurotoxin-resistant pathway (TeNT-resistant). The overwhole resulting effect of such N-terminal fragments on the properties of said cells is to inhibit their membrane traffic activity, *i*.*e*. the transport of components to its plasma membrane, notably through vesicular transport. Conversely, the TI-VAMP fragments which are deleted from their N-terminal domain (in so far that their coiled coil motif activity is appropriately preserved, *i*.*e*. said ΔNter fragments can still bind to at least one of their t-SNARE) are herein shown to exert stimulating effects on the membrane traffic activity of the cells into which they have been transfected : they are capable of stimulating their fusion functions, and in a particular aspect, their exocytic functions. Conversely to Nter fragments, such ΔNter fragments are herein shown to be capable of stimulating the formation of complexes involving VAMP, and notably complexes involving a VAMP such as TI-VAMP and at least one of its t-SNARE (SNAP 25, SNAP23, syntaxin1, syntaxin3). In fact, such ΔNter fragments may stimulate any cell function which involves a tetanus neurotoxin-resistant pathway (TeNT-resistant). The overwhole resulting effect of such ΔNter fragments on the properties of said cells is to inhibit their membrane traffic activity, *i*.*e*. the transport of components to its plasma membrane, notably through vesicular transport.
The invention thus offers new means for controlling membrane traffic into a cells, and in particular for regulating fusion functions into a cell such as exocytic functions, and notably vesicular transports of components to the plasma membrane. The means of the invention thus allow the regulation of very fundamental functions and properties of any cell that express a VAMP such as TI-VAMP. In this respect, the skilled persons can envisage and perform from the teaching of the invention a very wide range of applications, and indeed any applications involving the regulation of a traffic membrane and/or of a TeNT-resistant pathway. This notably includes the positive and negative control of neurite outgrowth and of cell motility, this latter application being of special interest in the case of metastasis-forming cells.

A particular aspect of the invention indeed more precisely relates to neurite outgrowth control: the present invention shows for the first time that SNARE-mediated vesicular transport is essential to neurite outgrowth, *i*.*e*. to axonal and dendritic maturation and differentiation. This is the first report of TI-VAMP mediating neurite outgrowth. Elongation of axon and dendrites, so-called neurite outgrowth, is a crucial event in neuronal differentiation and maturation ; and thus in the development of the nervous system. Membrane traffic in dendrites is also of importance for synaptic plasticity and memory.
It is herein further demonstrated that over-expression of a Nter fragment (such as SEQ ID N°2 or N°20) inhibits neurite outgrowth as strongly as botulinum neurotoxin (BoNT E), a neurotoxin which is known to abolish the expression of SNAP 25, the now-identified t-SNARE partner of TI-VAMP in cells such as neuronal cells. It is also observed that such Nter fragments inhibit the formation of TI-VAMP/SNAP25 complexes in neuronal cells, and that the reverse effects are induced by △Nter fragments of a VAMP such as TI-VAMP.

The above-described results indeed demonstrates that TI-VAMP-mediated vesicular transport is essential for neurite outgrowth. Expression of the N-terminal domain of TI-VAMP inhibits neurite outgrowth as strongly as BoNT E, which abolishes the expression of SNAP25, a plasma membrane SNARE partner of TI-VAMP. In the contrary, activation of neurite outgrowth and increased SNARE complex formation were observed when the N-terminus deletion mutant of TI-VAMP was expressed in PC12 cells.

A main conclusion from our work is that TI-VAMP is involved in neurite outgrowth in neuronal cells such as PC12 cells. Our findings that TI-VAMP interacts with SNAP25 in PC12 cells and in the brain is consistent with the involvement of SNAP25 in neurite outgrowth. The TI-VAMP-dependent vesicular transport mediating neurite outgrowth in PC12 cells likely corresponds to the outgrowth of axons and dendrites in developing neurons. Indeed, TI-VAMP concentrates in the leading edge of axonal and dendritic growth cones of hippocampal neurons in primary culture. In support of this conclusion, preliminary experiments have shown a decreased number of neurites in young hippocampal neurons, which were micro-injected with anti-TIVAMP antibodies. Neurite outgrowth may be also very active in differentiated neurons because it may participate to post-synaptic morphological changes related to plasticity and learning. A role for SNAP25 in neuronal plasticity and learning has been proposed. Therefore, the TI-VAMP and SNAP25 dependent vesicular transport mechanism described here could also mediate activity-dependent exocytosis involved in dendrite elongation and post-synaptic receptor expression at the plasma membrane in mature neurons. This could account for the distribution of TI-VAMP containing vesicles throughout the dendrites and of SNAP25 in the dendritic plasma membrane of mature neurons. According to our present results, the proteic and lipidic map of TI-VAMP vesicular compartment is likely to identify factors, which are important for neurite elongation both in developing and mature neurons. The purification of TI-VAMP vesicular compartment allows to determine which other proteins are involved in this pathway, particularly rab proteins that have been shown to play a role in neurite outgrowth.

We found that the cytoplasmic domain of TI-VAMP, which comprises the N-terminal domain plus the R-SNARE motif, had no effect on neurite outgrowth whereas the N-terminal domain alone strongly inhibited it. This demonstrates that the full cytoplasmic domain is inactive *in vivo.* The coiled-coiled domain of TI-VAMP bound more efficiently SNAP25 than the cytoplasmic domain by overlay assay. Therefore, our observations favour a model in which the N-terminal domain of TI-VAMP acted as an intramolecular inhibitor of the R-SNARE motif preventing it from forming SNARE complexes. Hence, identifying the signal transduction pathway(s) and factors, able to activate TI-VAMP, will be of crucial importance to further understand how neurite outgrowth is controlled.

Finally, our finding that the N-terminal domain of TI-VAMP plays an important function in the control of neurite outgrowth, suggests that this protein is a potential target of pharmacological agents that could modulate the activity of TI-VAMP by releasing the inhibition of this domain. Such agents could be used to specifically activate TI-VAMP mediated exocytosis thus, stimulate neurite outgrowth. Once identified, such drugs could be used in the treatment of nerve traumatisms such as spinal cord injury.

### EXEMPLE 2 : SEQ ID N°2 or N°20 (Nter) inhibits the motility of tumor cells

Cells such as tumor cells or the MDCK cell line (Mardin Darby Canine Kidney) can be used for transfection as above-described in example 1 so as to make them express a Nter polypeptide (M¹ to N¹²⁰ of TI-VAMP, of the SEQ ID N°1 and N°2 invention, a "deleted" polypeptide of the invention (M¹⁰² to the end of human TI-VAMP, SEQ ID N°3 and N°4), or the complete TI-VAMP sequence (SEQ ID N°5 and N°6).
These transfected cells can thus be placed into contact with a migration inductor such as a growth factor (*e*.*g*. HGF -Hepatocyte Growth Factor- for MKCK), and the differences in cell migration for each treatment can be observed video-microscopy and/or confocal microscopy.
When appropriate, or desired, *in vivo* observations can be performed.

### EXEMPLE 3:

Appropriate formulations for drugs of the invention notably comprise tablet and injection solution, spray for chemical or peptidic products, and comprise liposome and virus for DNA products.

### EXAMPLE 4: Expression of Nter and ΔNter in neurons via electroporation or Adeno-associated virus (Aav) infection

We expressed the amino-terminal domain and the form of TIVAMP deleted at its amino-terminus in neurons to assay for the role of TIVAMP in the outgrowth of axons and dendrites. We took advantage of new techniques for transfecting neurons, the electroporation of mouse embryonic brains, the infection of cortical-striatal neurons in primary culture with recombinant adeno-associated virus (Aavs) as well as the transfection mediated by calcium phosphate of primary hippocampal neurons to show that the expression of only the amino-terminal domain of TIVAMP blocks axonal and dendritic outgrowth, and alters the localization of the excitatory amino acid carrier 1 (EAAC1), a dendritic plasma membrane protein that is expressed early in neuronal development (Coco et al. 1997, Eur. J. Neurosci. 9: 1902-1910 ; Verderio et. al. 1999, Cellular and Molecular Life Science 55: 1448-1462). Expression of the form of the protein deleted at its amino-terminus led to an increase in axonal length.

### Experimental procedures

### Antibodies and Clones

Rabbit serums (TG11 and TG16, Galli et al. 1998, Mol. Biol. Cell 9:1437-1448 ; Lafont et al. 1999 Proc. Natl. Acad. Sci. USA 96:3734-3738) directed against TIVAMP were purified by affinity chromatography in a column loaded with a 6xHis-fusion protein of the C-terminal SNARE motif of TIVAMP (from residue E119 to K188). For the micro-injection experiments the affinity-purified TG11/16 or control rabbit IgGs at lmg/ml were dialyzed against PBS. A polyclonal antibody directed against GFP was generated in rabbit and affinity purified over recombinant GST-coupled GFP. Mouse monoclonal antibody directed against synaptobrevin 2 (clone 69.1) is obtainable from Max Planck Inst., Goettingen, FRG. Antibodies against EAAC1 and GluR1 were used as described in Coco et al. 1997, Eur. J. Neurosci. 9: 1902-1910. The cDNA of human TIVAMP and the GFP-fusion constructs have been described in the above example 1.

### Electroporation of mouse embryonic brain

Cortex were dissected out from mouse E13 embryos in PBS, 0.6 glucose (PBS-G). Plasmids (2µg/µl) were co-injected with 0.05% Fast-Green (Sigma, St Louis, MO) using a glass capillary needle. Electroporation was performed by 5 pulses (50 V, 50ms) with a T-820 apparatus (BTX, San Diego, CA) using tweezer electrodes (TR Tech Co Ltd, Tokyo, Japan). After electroporation cells were dissociated with PBS-G containing trypsin. Dissociated cells were plated in matrigel-coated glass coverslips in chemically defined medium as described (Mainguy et al. 2000, Nat. Biotechnol. 18(7): 746-749) supplemented with 10% FCS. After the indicated times cells were fixed with 4% PFA and either mounted with Vectashield-DAPI (4',6-diamidino2-phenylindole) (Vector Lab., Inc. Burlingame, CA) for observation of direct GFP-signal or permeabilized with 0.3% Triton X-100 and processed for immunofluorescence according to standard techniques (Coco et al. 1999, J. Neurosci. 19:9803-9812).

### Aav vectors construction, production, purification and titration

rAAV-CMV-GFPTIVAMP, rAAV-CMV-GFP-NtermTIVAMP, and rAAV-CMV-GFP-DTIVAMP vectors were respectively obtained from the pCR3.1GFPTIVAMP, pCR3.1GFPNtermTIVAMP, pCR3.1GFPDTIVAMP, plasmids, harboring the corresponding transgene and the pGG2 AAV plasmid. The latter plasmid is derived from the pSUB201 plasmid (obtainable from ATCC), where the expression is driven by hCMV promoter and stabilized by the SV40 late polyA and a chimeric intron composed of the 5' donor splice site of the first intron of the human beta globin gene (hBB) and the 3' acceptor splice site of the intron of an immunoglobulin gene (IgG) heavy chain variable region. First, GFPTIVAMP, GFPNtermTIVAMP, GFPDTIVAMP sequences were PCR-amplified by using specific primers and the high fidelity pfu turbo polymerase (Stratagene, La Jolla, CA ) and further digested by NheI restriction enzyme at the 3' end. These fragments were purified from agarose gel by using the geneclean kit (BIO101, Vista, CA) according to the manufacturer's procedure. Secondly, the pGG2 plasmid was cut by NheI and EcoRV enzymes to add the PCR-amplified cDNAs. The correct orientation of the inserted sequences were checked by DNA sequencing analysis and agarose gel electrophoresis. Large scale production and purification of vectors were performed by using the triple transfection of 293 cells (obtainable from ATCC), followed by CsC1 density gradients purification, as previously described (Xiao 1998, J. Cell. Biol. 143: 1077-1086). The infectious particle concentration is determined by a variation of the procedure previously described (Salvetti 1998, Hum. Gene Ther. 9(5): 695-706).

### Adeno-associated viral infection of neurons

Cortical and striatal neurons were prepared from rat E16 embryos according to standard techniques in the art. After dissociation neurons were plated in collagen-coated glass coverslips in chemically defined medium as above. Five hours after plating cells were infected overnight with the described Aavs at a MOI of 100 in a final volume of 50µl. The day after the Aavs were removed and cells were kept in regular medium for the indicated periods of time. The direct GFP signal from the Aav-encoded proteins could be detected 3 days after infection, however, to facilitate detection of the infected neurons for subsequent quantitation, cells were fixed/permeabilized as described above and stained with anti-GFP antibodies.

### Transfection of hippocampal neurons with calcium phosphate

Calcium phosphate crystals were prepared as described in Maniatis et al. 1982 (Molecular Cloning: a laboratory manual (Cold Spring Harbor, New York: Cold Spring Harbor Laboratory, of which content is herein incorporated by reference). For transfection, neurons were placed in medium conditioned by cortical astrocytes for at least 15 hours. Calcium phosphate crystals were left for four hours, the cells were then washed accurately with Krebs-Ringer solution and transferred in their previous medium.

### Antibody micro-injection in hippocampal neurons

Cells used in micro-injection experiments were seeded onto etched coverslips to facilitate localization of injected cells. During micro-injection experiments cells were maintained in Krebs-Ringer solution. Micro-injections were performed in blind using a solution containing 1mg/ml of an affinity-purified rabbit antibody directed against TIVAMP or control IgGs. Neurons were co-injected with 1 mg/ml dextran-FITC conjugated IgG (Sigma) to identify injected cells. Micro-injections were performed using the Eppendorf 5242 micro-injector. Commercial glass micro-capillaries with an outlet diameter of 0.5 +/- 0.2 mm (Femptotips; Eppendorf, Hamburg, FRG) were used.

### Quantification of axonal and dendritic length in hippocampal neurons

Randomly chosen fields were taken with a BioRad MRC-1024 Confocal Microscope equipped with a LaserSharp 3.2 software. Acquired images were processed and quantitatively analyzed with NIH Image 1.62 software from National Institute of Health, resulting in the analysis of between 40 and 60 GFP-positive cells, for each condition and for each independent experiment. For immunocytochemistry, neurons were permeabilized with 0.3% Triton X-100 and processed for immunofluorescence as described (Coco et al. 1999, J. Neurosci. 19:9803-9812).

### Quantification of axonal length in cortico-striatal neurons

Randomly chosen fields were taken with a MicroMax CCD camera (Princeton Instruments), resulting in the analysis of between 10 and 50 (in the electroporation experiments) or between 25 and 200 (in the Aav experiments) GFP-positive cells, for each condition and for each independent experiment. Quantification of axonal length was done using the Metamorph software (Princeton Instruments). Double immunofluorescence with neuronal markers was performed in order to verify exclusively quantification of neuronal cells. The obtained data were analyzed for their statistical significance with Sigma Stat (SPSS, Inc.).

### Quantification of Nter-TIVAMP-induced cell death

After electroporation of embryonic E13 brains cells were dissociated and cultured in the absence or presence of 200µM zVAD (Calbiochem, La Jolla, CA) to inhibit caspases thus apoptosis. After 24h all the green-fluorescent neuronal and non-neuronal cells remaining were scored.

### Results

### Expression of the amino-terminal domain of TIVAMP inhibits neuronal differentiation.

To investigate the role of TIVAMP in neuronal differentiation we expressed GFP and GFP fused to the amino-terminal domain of TIVAMP (GFP-Nter-TIVAMP), using a calcium phosphate-based transfection method, in E18 rat hippocampal neurons that were cultured in the presence of feeding glial cells (cf. Figure 9). Neurons were transfected at 1 div or 4 div (div = days in vitro) and examined 24 hours later. Dendritic function was significantly altered in hippocampal neurons expressing GFP-Nter-TIVAMP because a reduced localization of the glutamate transporter EAAC1 was seen in the dendrites of these cells compared with cells expressing only GFP (Figure 9). Quantification of dendrites positive for EAAC1 showed that far fewer of them were from neurons expressing GFP-Nter-TIVAMP than from neurons expressing only GFP (Figure 10A). We did not observe any effect on the localization of the AMPA receptor sub-unit GluRl (Figure 9). This lack of effect shows that only a subset of transport pathways are inhibited by the expression of GFP-Nter-TIVAMP. Interestingly, dendrites of neurons expressing GFP-Nter-TIVAMP were fewer in number and shorter than those neurons expressing GFP, after 2 div and 5 div; the strongest effect was after 2 div (Figure 10B, 10C). In comparison, the inhibitory effect of the expression of GFP-Nter-TIVAMP on axonal outgrowth appeared stronger than the effect on dendritic outgrowth, when only the lengths of the processes were compared (compare Figure 10B with 10D). The involvement of TIVAMP in dendritic outgrowth was further confirmed by the finding that micro-injection of an affinity-purified rabbit antibody directed against this protein into 1 div old hippocampal neurons from embryonic E18 rats but not of control rabbit IgGs resulted in a strong reduction of the number of dendrites (Figure 10E).

Our observation that the inhibitory effect GFP-Nter-TIVAMP was smaller when the transfection was performed in 4 div hippocampal neurons compared to 1 div neurons prompted us to study axonal and dendritic outgrowth in embryonic neurons of earlier stages. For doing so, we electroporated intact embryonic brains from E13 mice, then dissociated the cortices and striata and cultured neurons and astrocytes. The cells were plated, cultured and observed after 1 to 3 div. Using this approach, cells expressing GFP were abundant at both time points. The development of neurons expressing GFP was indistinguishable from that of non-transfected neurons and normal axonal and dendritic outgrowth was observed. However, after 1 div, we could find only few cells expressing GFP-Nter-TIVAMP and after 3 div, almost none were visible (Figure 11A). Presumably expression of GFP-Nter-TIVAMP at early times during development blocked neuronal differentiation and rapidly induced neuronal cell death. To test this presumption, we treated the cells with benzyloxycarbonyl-Val-Ala-Asp-(OMe)-fluoromethylketone (zVAD), a broad spectrum inhibitor of caspase (Polverino and Patterson 1997, J. Biol. Chem. 272(11): 7013-7021), shortly after plating. Cells treated with zVAD and expressing GFP-Nter-TIVAMP survived after 1 div but differentiation, as assessed by axonal and dendritic outgrowth, was severely impaired when compared with the corresponding cells expressing GFP alone (Figure 11A). Quantification of survival showed that zVAD reversed the pro-apoptotic effect induced by the expression of GFP-Nter-TIVAMP (Figure 11B). The neurons treated with zVAD and expressing GFP-Nter-TIVAMP surviving after 1 div showed a significant reduction in axonal length relative to cells expressing GFP alone (35 µm compared to 60 µm) (Figure 11C) and only 16% of their axons were longer than 50 µm compared with 62% in the case of GFP. This result suggests that the deleterious effect of the expression of Nter-TIVAMP was due, at least in part, to inhibition of axonal and dendritic outgrowth. The electroporation of intact brain, however, led to high level of expression of the transgenes and the apotototic effect could be due to nonspecific toxicity at high intracellular concentration, even though the control cells seemed normal. Therefore, we constructed recombinant Adeno-associated virus (Aavs) (Slack and Miller 1996, Curr. Opin. Neurobiol. 6(5)576-583; Du et al. 1996 Gene Ther. 3(3): 254-261) expressing GFP or GFP-Nter-TIVAMP and used them to infect cortico-striatal neurons. In this case also, the expression of GFP-Nter-TIVAMP resulted in strong inhibition of axonal and dendritic outgrowths, after 1, 2 and 3 div (Figure 11D). Staining with DAPI showed that the DNA of cells expressing GFP-Nter-TIVAMP but not the DNA of cells expressing GFP was condensed and fragmented. This effect was seen already in a majority of GFP-Nter-TIVAMP-expressing neurons after 1 div but affected virtually all of the cells after 3 div (representative cells are depicted in Figure 11D). Thus, the expression of GFP-Nter-TIVAMP, mediated by the corresponding recombinant Aav, resulted in neuronal cell death also, in spite of the fact that infection with Aav induced a much lower level of expression than electroporation. Consequently, this effect was independent of the method of transfection. Altogether, these results demonstrated that that TIVAMP is one of the proteins essential for both axonal and dendritic outgrowth.

### A constitutively active form of TIVAMP enhances axon outgrowth.

We have previously found that the expression of a form of TIVAMP from which the amino-terminal domain has been deleted (GFP-ΔNter-TIVAMP) stimulated neurite outgrowth in PC12 cells (see the above example 1). Because expression of GFP-Nter-TIVAMP blocked both dendritic and axonal outgrowths in neurons, we asked whether or not expression of GFP-ΔNter-TIVAMP had any effect on these processes. Transfection of plasmids expressing GFP-ΔNter-TIVAMP by electroporation of intact E13 murine brains greatly stimulated axonal outgrowth of neurons in primary culture (Figure 12, 13A), and rat cortico-striatal neurons infected with Aavs producing expression of GFP-ΔNter-TIVAMP also showed increased axonal outgrowth (Figure 12, 13B). None of these effects were seen in control cells upon expression of GFP or GFP-TIVAMP. These stimulatory effects could be seen at 1, 2, 3 or 6 div but the strongest effects were observed after 3 and 6 div in the case of Aav-treated neurons. Most remarkably, expression of GFP-ΔNter-TIVAMP increased fourfold the percentage of axons longer than 300 µm after 6 div (Figure 13B). The expression of full-length TIVAMP had no effect relative to the expression of GFP (Figures 13A, 13B). We did not observe any significant effect of the expression of GFP-△Nter-TIVAMP on dendritic length or on the number of dendrites per cell in any of the three models we have used.

Removal of the amino-terminal extension of TIVAMP produces a molecule of protein that has a structure typical of brevins. It was possible that GFP-ΔNter-TIVAMP has lost important targeting information and behaves as synaptobrevin 2 because it does not reach its site of normal function. If this were the case, these results would not provide insight into the function of TIVAMP in axonal and dendritic outgrowth. Therefore, we studied the subcellular location of GFP-ΔNter-TIVAMP and synaptobrevin 2 in cortico-striatal neurons 6 div after infection with Aavs. GFP-△Nter-TIVAMP was found in cell bodies, dendrites, axon hillocks, all along the axon, and in varicosities (Figures 14A to 14E). We found that GFP-△Nter-TIVAMP did not co-localize with synaptobrevin 2. Interestingly, GFP-△Nter-TIVAMP densely localized at the leading edge of axons in the peripheral region of growth cones, a location devoid of synaptobrevin 2 (Figure 14E), as seen for the endogenous protein (Coco et al. 1999, J. Neurosci. 19: 9803-9812).

### Discussion

As demonstrated in example 1, TIVAMP is one of the proteins essential for neurite outgrowth in PC12 cells. In this example, it is directly confirmed that TIVAMP is essential for both dendritic and axonal outgrowth in neurons. Expression of the amino-terminal domain of TIVAMP inhibited axonal and dendritic outgrowth. Expression of a form of TIVAMP from which the amino-terminal domain has been deleted strongly enhanced axonal outgrowth in mouse cortical and striatal neurons but had no effect on dendritic outgrowth. The fact that the expression of these two proteins had opposite effects shows that the observed changes were not the result of the transfection itself but the identity of the proteins themselves.

On the contrary, the amino-terminal domain of TI-VAMP seems to inhibit their capacity to form trans-SNARE complexes (example 1). Preliminary results show that a chimera consisting in the fusion of the amino-terminal domain of TIVAMP with synaptobrevin 2 leads to a v-SNARE with a reduced capacity to form complexes with syntaxin 4 and SNAP23 in fibroblasts. Altogether, our results suggest a model in which TIVAMP would be less active and more controlled v-SNAREs than brevins. The amino-terminal domain of TIVAMP is unlikely to contain targeting signals because the localization of △Nter-TIVAMP is similar to that of the full length protein (Figures 12 and 14A-14E ; Coco et al. 1999, see reference supra). Moreover, the stimulatory effect on axonal outgrowth resulting from expression of △Nter-TIVAMP (Figures 13A, 13B) is likely to be specific for TIVAMP because ΔNter-TIVAMP does not co-localize with synaptobrevin 2 (Figures 14A-14E), in spite of the fact that it has a similar structure and high primary sequence similarity (Galli et al. 1998, Mol. Biol. Cell 9: 1437-1448).

Expression of Nter-TIVAMP inhibited neuronal differentiation (Figures 10A-10E) and led to neuronal cell death (Figures 11A-11D). This effect cannot be due to a general deleterious effect of this peptide because we have shown that Nter-TIVAMP inhibits neurite outgrowth but does not lead to cell death in PC12 cells (example 1). We found that apoptotic death occurred specifically in neurons and not in astrocytes.

It was recently shown that theShc site of TrkB controls both neuronal survival and axonal outgrowth by activating the PI3-kinase and MEK signaling pathways thus establishing a link between these two functions (Atwal et al. 2000, Neuron. 27(2): 265-277). Our results suggest that the apoptosis observed upon the expression of Nter-TIVAMP is a consequence of the inhibition of axonal and dendritic outgrowth.

The fact that expression of Nter-TIVAMP blocked both axonal and dendritic outgrowths (Figures 10A-10E and 11A-11D) indicates that both processes share common molecular mechanisms. The expression of Nter-TIVAMP in PC12 cells (see example 1) and in the neurons examined in this study had no effect on the structure of the Golgi apparatus, as seen by syntaxin 6 immunolabeling, or in the intracellular distribution of synaptobrevin 2 and axonal and dendritic cytoskeletal components such as tau and MAP-2, so it is unlikely to induce pleiotropic effects on membrane traffic or other cellular functions. As suggested by its localization at the leading edge of both axonal and dendritic growth cones, vesicles the fusion of which is promoted by TIVAMP could transport proteins that are required both for axonal and dendritic outgrowth (Coco et al. 1999, see reference supra). The effect of Nter-TIVAMP on the dendritic expression of EAAC1 (Figure 9), a protein that may play a role in synaptogenesis (Coco et al. 1997, Eur. J. Neurosci. 9: 1902-1910), but not on the expression of GluRl, a protein of the mature dendrite (Eshhar et al. 1993, Neurosci. 57(4): 943-964), suggests that TIVAMP could be involved in exocytosis of a very limited number of axonal and dendritic protins that are expressed at early stages of neuronal development. Our observation that expression of ΔNter-TIVAMP had no effect on dendritic outgrowth and that the expression of the Nter-TIVAMP had a higher efect on axons than on dendrites of hippocampal neurons (Figures 10A-10E) can be explained if dendritic outgrowth normally functions at maximal rate and thus cannot be further activated, at least under our conditions of culture. If this were so, the exocytosis mediated by TIVAMP would be regulated differently in axons and dendrites. This would be in agreement with several experiments showing that dendritic and axonal outgrowths are controlled by different signals (Prochiantz 1995, Neuron. 15: 743-746). An alternative possibility is that TIVAMP is primarily involved in axonal outgrowth and that dendritic outgrowth can proceed only when axonal outgrowth occurs normally. Indeed, our observations are reminiscent of recent work showing that amyloid precursor protein first appears in axons and is then transported to dendrites by transcytosis. Both amyloid precursor protein and TIVAMP have been found in rafts, so the hypothesis that TIVAMP would follow neuronal transcytosis is a fashionable one. It will now be important to characterize the proteins which control TIVAMP's mediated exocytosis. Specific axonal and dendritic factors are expected to regulate this pathway thus accounting for differential control of the growth rate of axons and dendrites in different types of neurons. Such factors may include rab proteins (Huber et al. 1995, Molecular & Cellular Biology 15: 918-924), GTPases of the Rac and Rho families (Nakayama et al. 2000, J. Neurosci. 20(14): 5329-5338) and kinesins (Terada and Hirokawa 2000, Curr. Opin. Neurobiol. 10(5): 566-573).

It has been shown that TIVAMP is involved in several membrane trafficking steps in different cell types. It mediates apical exocytosis in epithelial cells, degranulation in mast cells, and participates in the EGF degradative pathway. This study establishes its intimate involvement in axonal and dendritic outgrowth. An appealing hypothesis could be that, among other cargo proteins, vesicles controlled by TIVAMP could contain hydrolases. These enzymes could be involved in the processing of membrane proteins and/or they could fulfill a function once they are secreted. Secretion of certain hydrolases may be important for elongation of axons and pathfinding because they would allow for specific penetration of the extracellular medium by cleaving particular components of the basal lamina that would otherwise prevent elongation (McGuire and Seeds 1990, Neuron 4(4): 633-642 ; Seeds et al. 1990, Adv. Exp. Med. Biol. 265: 169-178). If this is the case, TIVAMP-containing vesicles would be routed to different target membranes depending on the cell type: to endocytic structures in the case of fibroblasts or to plasma membranes in the case of epithelial cells, mast cells and differentiating neurons. Such differences could also be correlated with different developmental stages. Identification of the content of these vesicles in neurons is expected to yield proteins that are important for axonal outgrowth and may suggest new strategies for the treatment of severe traumatic nerve injuries.

## Claims

1. Isolated polypeptide, the sequence of which is selected from the group consisting of a sequence corresponding to SEQ ID N°2, a sequence corresponding to SEQ ID N°20, the sequences corresponding to any conservative fragment of SEQ ID N°2, the sequences corresponding to any conservative fragment of SEQ ID N°20, the sequences corresponding to any conservative variant of SEQ ID N°2, the sequences corresponding to any conservative variant of SEQ ID N°20.

2. Isolated polypeptide, the sequence of which is selected from the group consisting of the SEQ ID N°6 sequence of which N-terminal domain has been deleted of at least one polypeptide according to claim 1.

3. Isolated polypeptide, the sequence of which is selected from the group consisting of SEQ ID N°4, the sequences corresponding to any conservative variant of SEQ ID N°4, and the sequences corresponding to any conservative fragment of SEQ ID N°4.

4. Product selected from the group consisting of the monoclonal antibodies capable of binding to a polypeptide according to claim 1, and the Fab, F(ab')₂, CDR fragments thereof.

5. Product according to claim 4, **characterized in that** it is capable under physiological conditions of inhibiting at least one of the biological properties a polypeptide according to claim 1 can show.

6. Isolated polynucleotide, the sequence of which codes for a polypeptide according to claim 1.

7. Isolated polynucleotide, the sequence of which is selected from the group consisting of a sequence corresponding to SEQ ID N°1, a sequence corresponding to SEQ ID N°19, the sequences corresponding to any conservative fragment of SEQ ID N°1, the sequences corresponding to any conservative variant of SEQ ID N°1, the sequences corresponding to any conservative fragment of SEQ ID N°19, the sequences corresponding to any conservative variant of SEQ ID N°19.

8. Isolated polynucleotide, the sequence of which codes for a polypeptide according to any one of claims 2-3.

9. Isolated polynucleotide, the sequence of which is selected from the group consisting of the SEQ ID N°5 sequences of which 5' domain has been deleted from at least one polynucleotide according to any one of claims 6-7.

10. Isolated polynucleotide, the sequence of which is selected from the group consisting of a sequence corresponding to SEQ ID N°3, the sequences corresponding to any conservative fragment of SEQ ID N°3, the sequences corresponding to any conservative variant of SEQ ID N°3.

11. Isolated polynucleotide, the sequence of which codes for a product according to any one of claims 4-5.

12. Transfection vector, which comprises at least one polynucleotide according to claim 6 or 7.

13. Adeno-associated virus, comprising at least one polynucleotide according to claim 6 or 7.

14. Transfection vector, which comprises at least one polynucleotide according to any one of claims 8-11.

15. Adeno-associated virus, comprising at least one polynucleotide according to any one of claims 8-11.

16. Genetically engineered cell comprising at least one element selected from the group consisting of the polynucleotides according to claim 6, the polynucleotides according to claim 7, the transfection vectors according to claim 12, the adeno-associated virus according to claim 13.

17. Genetically engineered cell comprising at least one element selected from the group consisting of the polynucleotides according to claims 8-11, the transfection vectors according to claim 14, the adeno-associated virus according to claim 15.

18. Pair of oligonucleotides **characterized in that** it is capable under standard PCR conditions to amplify at least one polynucleotide according to claim 6 or 7.

19. Pair of oligonucleotides of which respective sequences correspond to SEQ ID N°11 and SEQ ID N°12.

20. Pair of oligonucleotides **characterized in that** it is capable under standard PCR conditions to amplify at least one polynucleotide according to any one of claims 8-10.

21. Pair of oligonucleotides of which respective sequences correspond to SEQ ID N°15 and SEQ ID N°16.

22. Isolated polynucleotide obtainable by using on a polynucleotide population at least one pair of oligonucleotides according to claim 18 or 19.

23. Isolated polynucleotide obtainable by using on a polynucleotide population at least one pair of oligonucleotides according to claim 20 or 21

24. Pharmaceutical composition comprising at least one product selected from the group consisting of the polypeptides according to claim 1, the polynucleotides according to claims 6, 7, 22, the transfection vectors according to claim 12, the adeno-associated virus according to claim 13, and the cells according to claim 16.

25. Pharmaceutical composition comprising at least one product selected from the group consisting of the polypeptides according to claims 2-3, the polynucleotides according to claims 8-11, 23, the transfection vectors according to claim 14, the adeno-associated virus according to claim 15, and the cells according to claim 17.

26. Method for identifying a pharmaceutical agent capable of stimulating a cell function selected from the group consisting of the cell functions involving a membrane traffic, the cell functions involving a TeNT-resistant pathway, the cell functions involving the formation of complexes with TI-VAMP, the cell functions involving at least one TI-VAMP, the cell functions involving a fusion, or an exocytic event, the cell functions involved in neurite outgrowth, in neuronal maturation, in neuronal differentiation, in neuronal or dendritic viability, in memory abilility, and in learning capacity, **characterized in that** it comprises at least one step chosen the group consisting of:
- the identification of an agent that is capable under physiological conditions of blocking or diminishing the inhibition effect that is observed when the cytoplasm of a cell is placed into contact with at least one product selected from the group consisting of the polypeptides according to claim 1, the polynucleotides according to claim 6, 7, 22, the transfection vectors according to claim 12, the adeno-associated virus according to claim 13, the cells according to claim 16, under conditions appropriate to cell membrane trafficking,
- the identification of an agent that is capable under physiological conditions to compete with a product according to any one of claims 4-5 for binding to at least one polypeptide according to claim 1,
- the identification of an agent that is capable under physiological conditions of stimulating the formation of complexes involving at least one polypeptide according to any one of claims 2-3, and at least one t-SNARE,
- the identification of an agent that is capable under physiological conditions of exerting an additional or synergic effect on the cell function observed when the cytoplasm of a cell is placed under conditions appropriate to cell membrane trafficking, into contact with at least one product selected from the group consisting of the polypeptides according to claims 2-3, the products according to claims 4, 5, the polynucleotides according to claims 8-11, 23, the transfection vectors according to claim 14, the adeno-associated virus according to claim 15, the cells according to claim 17,
- the identification of an agent that is capable under physiological conditions of preventing the formation of complexes between compounds which comprise at least one polypeptide according to claim 1, or of disrupting or destabilizing such complexes.

27. Method for identifying a pharmaceutical agent capable of inhibiting a cell function selected from the group consisting of the cell functions involving a membrane traffic, the cell functions involving a TeNT-resistant pathway, the cell functions involving the formation of complexes with TI-VAMP, the cell functions involving at least one TI-VAMP, the cell functions involving a fusion, or an exocytic event, the cell functions involved in cell motility, the cell functions involved in the formation of metastasis, **characterized in that** it comprises at least one step selected from the group consisting of:
- the identification of an agent that is capable under physiological conditions of stimulating the inhibition effect observed when the cytoplasm of a cell is placed under conditions appropriate to cell membrane trafficking, into contact with at least one product selected from the group consisting of the polypeptides according to claim 1, the polynucleotides according to claim 6, 7, 22, the transfection vectors according to claim 12, the adeno-associated virus according to claim 13, the cells according to claim 16,
- the identification of an agent that is capable under physiological conditions of inhibiting the formation of complexes involving at least one polypeptide according to any one of claims 2-3, and at least one t-SNARE,
- the identification of an agent that is capable under physiological conditions of exerting an inhibitory effect on a cell function that is observed when the cytoplasm of a cell is placed into contact, under conditions appropriate to cell membrane trafficking, with at least one product selected from the group consisting of the polypeptides according to claims 2-3, the products according to claims 4-5, the polynucleotides according to claims 8-11, 23, the transfection vectors according to claim 14, the adeno-associated virus according to claim 15, the cells according to claim 17,
- the identification of an agent that is capable under physiological conditions to lyse a product according to claim 5,
- the identification of an agent that is capable under physiological conditions of stimulating the formation of complexes between compounds which comprise at least one polypeptide according to claim 1, or of stabilizing such complexes, or of preventing the disruption of such complexes.

28. The use of at least one product selected from the group consisting of the polypeptides according to claim 1, the polynucleotides according to claim 6, 7, 22 the transfection vectors according to claim 12, the adeno-associated virus according to claim 13, the cells according to claim 16, the pharmaceutical agents obtainable by the method of claim 27, for the production of a drug intended for at least one effect selected from the group consisting of inhibiting a cell membrane traffic, inhibiting a vesicular transport, inhibiting a function involving a TeNT-resistant pathway, inhibiting a function involving at least one TI-VAMP, inhibiting the formation of complexes involving at least one TI-VAMP and at least one t-SNARE, inhibiting a cell motility, inhibiting the motility of cells susceptible of forming metastasis.

29. The use of at least one product selected from the group consisting of the polypeptides according to any ones of claims 2-3, the products according to any one of claims 4-5, the polynucleotides according to any one of claims 8-11, 23, the transfection vectors according to claim 14, the adeno-associated virus according to claim 15, the cells according to claim 17, the pharmaceutical agents obtainable by the method of claim 26, for the production of a drug intended for at least one effect selected from the group consisting of stimulating a cell membrane traffic, stimulating a vesicular transport, stimulating a function involving a TeNT-resistant pathway, stimulating a function involving at least one TI-VAMP, stimulating the formation of complexes involving at least one TI-VAMP and at least one t-SNARE, stimulating axonal and/or dendritic outgrowth, stimulating neuronal maturation, stimulating neuronal differentiation, preventing or decreasing neuronal apoptosis, stimulating memory and/or learning capacity, curing and/or palliating and/or preventing spinal cord trauma, curing and/or palliating and/or preventing neuro-degenerative disorders, curing and/or palliating and/or preventing sclerosis.

30. Method for the diagnostic an undesired state, and/or for assessing the efficiency of a medical treatment, and/or for assessing the evolution of an undesired state, **characterized in that** it comprises at least one step selected from the group consisting of:
- detecting in a biological sample that a product selected from the group consisting of the polypeptides according to claims 2-3, the products according to claims 4-5, the polynucleotides according to claims 8-11, 23, the transfection vectors according to claim 14, the adeno-associated virus according to claim 15, the cells according to claim 17, is present in a quantity or concentration significantly different from the standard level, or
- detecting in a biological sample a level of expression for a polypeptide according to claim 1 significantly different from the standard level, or
- detecting in a biological sample that the quantity or concentration of complexes involving compounds which comprise at least one polypeptide according to claim 1, is significantly different from the standard level.

31. A method for identifying a compound capable of acting as a biological effector of TI-VAMP, **characterized in that** it comprises:
- the detection of a compound which is capable under physiological conditions of binding to a polypeptide according to claim 1,
- the detection of a compound which is capable under physiological conditions of diminishing the inhibition effect that is observed when the cytoplasm of a cell expressing TI-VAMP is placed into contact with at least one product selected from the group consisting of the polypeptides according to claim 1, the polynucleotides according to claim 6, 7, 22, the transfection vectors according to claim 12, the adeno-associated virus according to claim 13, the cells according to claim 16, under conditions appropriate to cell membrane trafficking, the detection of a compound which is capable under physiological conditions of exerting an additional or synergic effect on the cell function that is observed when the cytoplasm of a cell expressing TI-VAMP is placed into contact with at least one product selected from the group consisting of the polypeptides according to claims 2-3, the products according to claims 4-5, the polynucleotides according to claims 8-11, 23, the transfection vectors according to claim 14, the adeno-associated virus according to claim 15, the cells according to claim 17, under conditions appropriate to cell membrane trafficking.
